# EUROPEAN PATENT APPLICATION

(11) **EP 3 444 272 A1**
(43) Date of publication of application: **20.02.2019**
(21) Application number: 17306073.2
(22) Date of filing: 17.08.2017
(51) Int. Cl.: C07K 16/18, A61K 33/24

(54) **TREATMENT OF CK8 POSITIVE CANCERS IN RELATION WITH K-RAS GENE STATUS**

(71) Applicant: International-Drug-Development-Biotech, 69007 Lyon (FR)
(72) Inventor: VERMOT-DESCROCHES, Claudine Brigitte Fernande, 69570 Dardilly (FR); LAVOCAT, Emilie, 69007 Lyon (FR); DOERFLINGER, Franck, 68700 Cernay (FR)
(74) Representative: Lavoix

(57) **Abstract**

The invention relates to the use of an anti-CK8 antibody alone or in combination therapy (with another antibody and/or chemotherapeutic agent) to (1) treat solid tumours expressing CK8 having wild-type K-Ras (not mutated as disclosed herein) and (2) treat solid tumours expressing CK8 having K-Ras mutation as disclosed herein. The invention also relates to the use of anti-CK8 antibodies having internalizing property, allowing to deliver cytotoxic agent coupled to antibody for the treatment of CK8 positive solid tumours, having wild-type K-Ras (not mutated as disclosed herein) or having K-Ras mutation as disclosed herein.

SEQUENCE LISTING

<110>  INTERNATIONAL-DRUG-DEVELOPMENT-BIOTECH

<120>  TREATMENT OF CK8 POSITIVE CANCERS IN RELATION WITH K-RAS GENE
       STATUS

<130>  BET17L3082

<160>  66

<170>  PatentIn version 3.5

<210>  1
<211>  336
<212>  DNA
<213>  Artificial Sequence

<220>
<223>  VL mD-A10

<400>  1
aacattgtta tgacccaggc cgcaccctct gtacctgtca ctcctggaga gtcagtatcc      60

atctcctgca ggtctagtaa gagtcttctg tatagtaatg gcaacactta tttgtattgg     120

ttcctgcaga ggccaggcca gtctcctcag cgcctgatat attatatgtc caaccttgcc     180

tcaggagtcc cagacaggtt cagtggcaga gggtcaggaa ctgatttcac actgagaatc     240

agtagagtgg aggctgagga tgtgggtgtt tattactgta tgcaaagtct agaatatcct     300

ttcacgttcg gtggaggcac caagctcgag atcaaa                               336


<210>  2
<211>  112
<212>  PRT
<213>  Artificial Sequence

<220>
<223>   VL  mD-A10

<400>  2

Asn Ile Val Met Thr Gln Ala Ala Pro Ser Val Pro Val Thr Pro Gly
1               5                   10                  15


Glu Ser Val Ser Ile Ser Cys Arg Ser Ser Lys Ser Leu Leu Tyr Ser
            20                  25                  30


Asn Gly Asn Thr Tyr Leu Tyr Trp Phe Leu Gln Arg Pro Gly Gln Ser
        35                  40                  45


Pro Gln Arg Leu Ile Tyr Tyr Met Ser Asn Leu Ala Ser Gly Val Pro
    50                  55                  60


Asp Arg Phe Ser Gly Arg Gly Ser Gly Thr Asp Phe Thr Leu Arg Ile
65                  70                  75                  80

Ser Arg Val Glu Ala Glu Asp Val Gly Val Tyr Tyr Cys Met Gln Ser
                85                  90                  95

Leu Glu Tyr Pro Phe Thr Phe Gly Gly Gly Thr Lys Leu Glu Ile Lys
            100                 105                 110

<210>  3
<211>  354
<212>  DNA
<213>  Artificial Sequence

<220>
<223>  VH mD-A10

<400>  3
gaagtgcagc tgttggagac tggaggaggc ttggtgcaac cgggggggtc acggggactc        60

tcttgtgaag gctcagggtt tacttttagt ggcttctgga tgagctgggt tcgacagaca       120

cctgggaaga ccctggagtg gattggagac attaattctg atggcagtgc aataaaatac       180

gcaccatcca taaaggatcg attcactatc ttcagagaca atgacaagag caccctgtac       240

ctgcagatga gcaatgtgcg atctgaggac acagccacgt atttctgtat cgcccattac       300

tccggtgggg ggtttgctta ctggggtcaa ggaacctcgg tcaccgtctc ctca             354

<210>  4
<211>  118
<212>  PRT
<213>  Artificial Sequence

<220>
<223>  VH mD-A10

<400>  4

Glu Val Gln Leu Leu Glu Thr Gly Gly Gly Leu Val Gln Pro Gly Gly
1               5                   10                  15

Ser Arg Gly Leu Ser Cys Glu Gly Ser Gly Phe Thr Phe Ser Gly Phe
            20                  25                  30

Trp Met Ser Trp Val Arg Gln Thr Pro Gly Lys Thr Leu Glu Trp Ile
        35                  40                  45

Gly Asp Ile Asn Ser Asp Gly Ser Ala Ile Lys Tyr Ala Pro Ser Ile
    50                  55                  60

Lys Asp Arg Phe Thr Ile Phe Arg Asp Asn Asp Lys Ser Thr Leu Tyr
65                  70                  75                  80

Leu Gln Met Ser Asn Val Arg Ser Glu Asp Thr Ala Thr Tyr Phe Cys

85                  90                  95

Ile Ala His Tyr Ser Gly Gly Gly Phe Ala Tyr Trp Gly Gln Gly Thr
            100                 105                 110

Ser Val Thr Val Ser Ser
        115

<210>  5
<211>  11
<212>  PRT
<213>  Artificial Sequence

<220>
<223>  CDR1 VL mD-A10

<400>  5

Lys Ser Leu Leu Tyr Ser Asn Gly Asn Thr Tyr
1               5                   10

<210>  6
<211>  3
<212>  PRT
<213>  Artificial Sequence

<220>
<223>  CDR2 VL mD-A10

<400>  6

Tyr Met Ser
1

<210>  7
<211>  9
<212>  PRT
<213>  Artificial Sequence

<220>
<223>  CDR3 VL mD-A10

<400>  7

Met Gln Ser Leu Glu Tyr Pro Phe Thr
1               5

<210>  8
<211>  8
<212>  PRT
<213>  Artificial Sequence

<220>
<223>  CDR1 VH mD-A10

<400>  8

Gly Phe Thr Phe Ser Gly Phe Trp
1               5

<210>  9
<211>  8
<212>  PRT
<213>  Artificial Sequence

<220>
<223>  CDR2 VH mD-A10

<400>  9

Ile Asn Ser Asp Gly Ser Ala Ile
1               5

<210>  10
<211>  11
<212>  PRT
<213>  Artificial Sequence

<220>
<223>  CDR3 VH mD-A10

<400>  10

Ile Ala His Tyr Ser Gly Gly Gly Phe Ala Tyr
1               5                   10

<210>  11
<211>  8
<212>  PRT
<213>  Artificial Sequence

<220>
<223>  CDR1 VH HzD-A10

<400>  11

Gly Phe Thr Phe Ser Ser Tyr Trp
1               5

<210>  12
<211>  8
<212>  PRT
<213>  Artificial Sequence

<220>
<223>  CDR2 VH HzD-A10

<400>  12

Ile Asn Ser Asp Gly Ser Ser Thr
1               5

<210>  13
<211>  11
<212>  PRT

<213>  Artificial Sequence

<220>
<223>   CDR1 VL HzD-A10

<400>  13

Lys Ser Leu Leu Tyr Ser Asn Gly Tyr Asn Tyr
1               5                   10


<210>  14
<211>  112
<212>  PRT
<213>  Artificial Sequence

<220>
<223>   VL  HzD-A10

<400>  14

Asp Ile Val Met Thr Gln Ala Pro Leu Ser Leu Pro Val Thr Pro Gly
1               5                   10                  15


Glu Pro Ala Ser Ile Ser Cys Arg Ser Ser Lys Ser Leu Leu Tyr Ser
            20                  25                  30


Asn Gly Tyr Asn Tyr Leu Tyr Trp Phe Leu Gln Lys Pro Gly Gln Ser
        35                  40                  45


Pro Gln Leu Leu Ile Tyr Tyr Met Ser Asn Leu Ala Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Val Gly Val Tyr Tyr Cys Met Gln Ser
                85                  90                  95


Leu Glu Tyr Pro Phe Thr Phe Gly Gln Gly Thr Lys Leu Glu Ile Lys
            100                 105                 110


<210>  15
<211>  118
<212>  PRT
<213>  Artificial Sequence

<220>
<223>  VH  HzD-A10

<400>  15

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Val Trp Val
        35                  40                  45

Ser Asp Ile Asn Ser Asp Gly Ser Ser Thr Lys Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Ala Glu Asp Thr Ala Val Tyr Tyr Cys
                85                  90                  95

Ile Ala His Tyr Ser Gly Gly Gly Phe Ala Tyr Trp Gly Gln Gly Thr
            100                 105                 110

Leu Val Thr Val Ser Ser
        115

<210>  16
<211>  354
<212>  DNA
<213>  Artificial Sequence

<220>
<223>  VH  HzD-A10

<400>  16
gaagtacaat tggtagaatc aggtggtggt ttggttcagc caggaggatc actgagactg      60

tcctgcgctg caagcggctt taccttctct agctactgga tgtcttgggt ccggcaagcc     120

ccagggaagg gactggtgtg ggtgagcgat attaatagtg acggctcttc tactaagtat     180

gctgatagtg tcaagggccg attcaccatc tcacgagaca acgccaagaa caccttgtac     240

ctccagatga actctttgag agctgaggat acagcagtgt attactgtat cgcccactac     300

tcagggggag gctttgctta ctggggtcaa ggcacactcg tgacagtctc ctct           354

<210>  17
<211>  336
<212>  DNA
<213>  Artificial Sequence

<220>
<223>  VL  HzD-A10

<400>  17
gatattgtaa tgactcaagc tccactctcc ttgcctgtaa ctcctggaga gcccgcttct      60

attagctgta ggagtagtaa aagcctgctt tacagtaatg gttacaatta cctgtactgg     120

tttttgcaga agcctggaca gtcaccccag ctcctcatct attatatgtc taacttggcc 180

agtggtgtcc cagaccgttt tagtggcagc ggctcaggca ccgactttac ccttaagatc 240

agccgagtcg aggctgaaga cgtaggagtg tactactgta tgcagagtct tgagtatcca 300

ttcaccttcg ggcagggcac caagctcgaa ataaag 336

<210> 18
<211> 330
<212> PRT
<213> Artificial Sequence

<220>
<223> CH of chD-A10

<400> 18

<210>  19
<211>  993
<212>  DNA
<213>  Artificial Sequence

<220>
<223>  CH of chD-A10

<400>  19
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtcgtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 20
<211> 107
<212> PRT
<213> Artificial Sequence

<220>
<223> CL of chD-A10

<400> 20

<210> 21
<211> 321
<212> DNA
<213> Artificial Sequence

<220>
<223>  CL of chD-A10

<400>  21
cgaactgtgg ctgcaccatc tgtcttcatc ttcccgccat ctgatgagca gttgaaatct      60

ggaactgcct ctgttgtgtg cctgctgaat aacttctatc ccagagaggc caaagtacag     120

tggaaggtgg ataacgccct ccaatcgggt aactcccagg agagtgtcac agagcaggac     180

agcaaggaca gcacctacag cctcagcagc accctgacgc tgagcaaagc agactacgag     240

aaacacaaag tctacgcctg cgaagtcacc catcagggcc tgagctcgcc cgtcacaaag     300

agcttcaaca ggggagagtg t                                               321


<210>  22
<211>  16
<212>  PRT
<213>  artificial sequence

<220>
<223>  CDR1 VL mD-A10

<400>  22

Arg Ser Ser Lys Ser Leu Leu Tyr Ser Asn Gly Asn Thr Tyr Leu Tyr
1               5                   10                  15


<210>  23
<211>  7
<212>  PRT
<213>  artificial sequence

<220>
<223>  CDR2 VL mD-A10

<400>  23

Tyr Met Ser Asn Leu Ala Ser
1               5


<210>  24
<211>  5
<212>  PRT
<213>  artificial sequence

<220>
<223>  CDR1 VH mD-A10

<400>  24

Gly Phe Trp Met Ser
1               5


<210>  25
<211>  3
<212>  PRT

<213>  artificial sequence

<220>
<223>  CDR1 VH mD-A10

<400>  25

Gly Phe Trp
1


<210>  26
<211>  17
<212>  PRT
<213>  artificial sequence

<220>
<223>  CDR2 VH mD-A10

<400>  26

Asp Ile Asn Ser Asp Gly Ser Ala Ile Lys Tyr Ala Pro Ser Ile Lys
1               5                   10                  15


Asp




<210>  27
<211>  9
<212>  PRT
<213>  artificial sequence

<220>
<223>  CDR3 VH mD-A10

<400>  27

His Tyr Ser Gly Gly Gly Phe Ala Tyr
1               5


<210>  28
<211>  5
<212>  PRT
<213>  artificial sequence

<220>
<223>  CDR1 VH Hz D-A10

<400>  28

Ser Tyr Trp Met Ser
1               5


<210>  29
<211>  3
<212>  PRT
<213>  artificial sequence

<220>

<223>  CDR1 VH Hz D-A10

<400>  29

Ser Tyr Trp
1



<210>  30
<211>  17
<212>  PRT
<213>  artificial sequence

<220>
<223>  CDR2 VH Hz D-A10

<400>  30

Asp Ile Asn Ser Asp Gly Ser Ser Thr Lys Tyr Ala Pro Ser Ile Lys
1               5                   10                  15


Asp




<210>  31
<211>  9
<212>  PRT
<213>  artificial sequence

<220>
<223>  CDR3 VH Hz D-A10

<400>  31

His Tyr Ser Gly Gly Gly Phe Ala Tyr
1               5



<210>  32
<211>  9
<212>  PRT
<213>  artificial sequence

<220>
<223>  CDR3 VH Hz D-A10

<400>  32

His Tyr Ser Gly Gly Gly Phe Ala Tyr
1               5



<210>  33
<211>  16
<212>  PRT
<213>  artificial sequence

<220>
<223>  CDR1 VL Hz D-A10

<400>  33

Arg Ser Ser Lys Ser Leu Leu Tyr Ser Asn Gly Tyr Asn Tyr Leu Tyr
1               5                   10                  15

<210>  34
<211>  7
<212>  PRT
<213>  artificial sequence

<220>
<223>  CDR2 VL Hz D-A10

<400>  34

Tyr Met Ser Asn Leu Ala Ser
1               5

<210>  35
<211>  336
<212>  DNA
<213>  Artificial sequence

<220>
<223>  VL mR022 (D-D6)

<400>  35
gatgttgtga tgacccagac tccactctcc ctgcctgtca gtcttggaga tcaagcctcc        60

atctcttgca gatctagtca gagccttgta cacagtaatg gaaacaccta tttacattgg       120

tacctgcaga agccaggcca gtctccaaag gtcctgatct acaaagtttc caaccgattt       180

tctggggtcc cagacaggtt cagtggcagt ggatcaggga cagatttcac actcaagatc       240

agcagagtgg aggctgagga tctgggagtt tatttctgct ctcaaagtac acatgttcca       300

ttcacgttcg gtggaggcac caagctcgag atcaaa                                 336

<210>  36
<211>  112
<212>  PRT
<213>  Artificial sequence

<220>
<223>  VL-mR022 (D-D6)

<400>  36

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Val Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Phe Thr Phe Gly Gly Gly Thr Lys Leu Glu Ile Lys
            100                 105                 110

<210>  37
<211>  360
<212>  DNA
<213>  Artificial sequence

<220>
<223>  VH-mR022 (D-D6)

<400>  37
gatgtgcagc ttcaggagtc gggacctggc ctggtgaaac cttctcagtc tctgtccctc      60

acctgcactg tcactggcta ctcaatcacc agtgattatg cctggaactg gatccggcag     120

tttccaggaa acaaactgga gtggatgggc tacataagct acagtggtcg cactagctac     180

aacccatctc tcaaaagtcg aatctctatc actcgagaca catccaagaa ccatttcttc     240

ctgcagttga attctgtgac tactgaggac acagccacat attactgtgc cccacttacg      300

acaggggtag gctatgctat ggactactgg ggtcaaggaa cctcggtcac cgtctcctca     360

<210>  38
<211>  120
<212>  PRT
<213>  Artificial sequence

<220>
<223>  VH-mR022 (D-D6)

<400>  38

Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Ser Tyr Ser Gly Arg Thr Ser Tyr Asn Pro Ser Leu
    50                  55                  60

Lys Ser Arg Ile Ser Ile Thr Arg Asp Thr Ser Lys Asn His Phe Phe
65                  70                  75                  80

Leu Gln Leu Asn Ser Val Thr Thr Glu Asp Thr Ala Thr Tyr Tyr Cys
                85                  90                  95

Ala Pro Leu Thr Thr Gly Val Gly Tyr Ala Met Asp Tyr Trp Gly Gln
            100                 105                 110

Gly Thr Ser Val Thr Val Ser Ser
        115                 120

<210>  39
<211>  336
<212>  DNA
<213>  Artificial sequence

<220>
<223>  VL-mR022 (D-F5)

<400>  39
gatgttgtga tgactcaaac tccactctcc ctgcctgtca gtcttggaga tcaagcctcc      60

atctcttgca gatctagtca gagcattgta catagtaatg gaaacaccta tttagaatgg     120

tacctgcaga aaccaggcca gtctccaaag ctcctgatct acaaagtttc caaccgattg     180

tctggggtcc cagacaggtt cagtggcagt ggatcaggga cagacttcac actcaagatc     240

agcagagtgg aggctgagga tctgggagtt tattactgct ttcaaggttc acttgttccg     300

ctcacgttcg gtggaggcac caagctcgag atcaaa                               336

<210>  40
<211>  112
<212>  PRT
<213>  Artificial sequence

<220>
<223>  VL-mR022 (D-F5)

<400>  40

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Ile Val His Ser
            20                  25                  30

Asn Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Leu Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser Leu Val Pro Leu Thr Phe Gly Gly Gly Thr Lys Leu Glu Ile Lys
            100                 105                 110

<210>  41
<211>  354
<212>  DNA
<213>  Artificial sequence

<220>
<223>  VH-mR022 (D-F5)

<400>  41
caggtgcagc ttaaggagtc gggacctggc ctggtggcgc cctcacagag cctgtccatc      60

acttgcactg tctctgggtt ttcattaacc agctatggtg tacactgggt tcgccagcct     120

ccaggaaagg gtctggagtg gctgggagta atatgggctg gtggaagcac aaattataat     180

tcggctctca tgtccagact gcgcatcagc aaagacaact ccaagagcca agttttctta     240

aaaatgaaca gtctgcaaac tgatgacaca gccatgtact actgtgccag aatctatggt     300

aactacggga ggtttgctta ctggggtcaa ggaacctcgg tcaccgtctc ctca           354

<210>  42
<211>  118
<212>  PRT
<213>  Artificial sequence

<220>
<223>  VH-mR022 (D-F5)

<400>  42

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Thr Ser Tyr
            20                  25                  30

Gly Val His Trp Val Arg Gln Pro Pro Gly Lys Gly Leu Glu Trp Leu
        35                  40                  45

Gly Val Ile Trp Ala Gly Gly Ser Thr Asn Tyr Asn Ser Ala Leu Met
    50                  55                  60

Ser Arg Leu Arg Ile Ser Lys Asp Asn Ser Lys Ser Gln Val Phe Leu

65                  70                  75                  80

Lys Met Asn Ser Leu Gln Thr Asp Asp Thr Ala Met Tyr Tyr Cys Ala
                85                  90                  95

Arg Ile Tyr Gly Asn Tyr Gly Arg Phe Ala Tyr Trp Gly Gln Gly Thr
            100                 105                 110

Ser Val Thr Val Ser Ser
        115

<210>  43
<211>  8
<212>  PRT
<213>  Artificial sequence

<220>
<223>  H-CDR1 D-F5

<400>  43

Gly Phe Ser Leu Thr Ser Tyr Gly
1               5

<210>  44
<211>  7
<212>  PRT
<213>  Artificial sequence

<220>
<223>  H-CDR2 D-F5

<400>  44

Ile Trp Ala Gly Gly Ser Thr
1               5

<210>  45
<211>  12
<212>  PRT
<213>  Artificial sequence

<220>
<223>  H-CDR3 D-F5

<400>  45

Ala Arg Ile Tyr Gly Asn Tyr Gly Arg Phe Ala Tyr
1               5                   10

<210>  46
<211>  11
<212>  PRT
<213>  Artificial sequence

<220>

<223>  L-CDR1 D-F5

<400>  46

Gln Ser Ile Val His Ser Asn Gly Asn Thr Tyr
1               5                   10


<210>  47
<211>  3
<212>  PRT
<213>  Artificial Sequence

<220>
<223>  L-CDR2 D-F5

<400>  47

Lys Val Ser
1


<210>  48
<211>  9
<212>  PRT
<213>  Artificial sequence

<220>
<223>  L-CDR3 D-F5

<400>  48

Phe Gln Gly Ser Leu Val Pro Leu Thr
1               5


<210>  49
<211>  8
<212>  PRT
<213>  Artificial sequence

<220>
<223>  H-CDR1 D-D6

<400>  49

Gly Tyr Ser Ile Thr Ser Asp Tyr
1               5


<210>  50
<211>  7
<212>  PRT
<213>  Artificial sequence

<220>
<223>  H-CDR2 D-D6

<400>  50

Ile Ser Tyr Ser Gly Arg Thr
1               5

<210>  51
<211>  13
<212>  PRT
<213>  Artificial sequence

<220>
<223>  H-CDR3 D-D6

<400>  51

Ala Pro Leu Thr Thr Gly Val Gly Tyr Ala Met Asp Tyr
1               5                   10


<210>  52
<211>  11
<212>  PRT
<213>  Artificial sequence

<220>
<223>  L-CDR1 D-D6

<400>  52

Gln Ser Leu Val His Ser Asn Gly Asn Thr Tyr
1               5                   10


<210>  53
<211>  9
<212>  PRT
<213>  Artificial sequence

<220>
<223>  L-CDR3 D-D6

<400>  53

Ser Gln Ser Thr His Val Pro Phe Thr
1               5


<210>  54
<211>  5
<212>  PRT
<213>  Artificial sequence

<220>
<223>  H-CDR1 D-F5

<400>  54

Ser Tyr Gly Val His
1               5


<210>  55
<211>  16
<212>  PRT
<213>  Artificial sequence

<220>

<223>  H-CDR2 D-F5

<400>  55

Val Ile Trp Ala Gly Gly Ser Thr Asn Tyr Asn Ser Ala Leu Met Ser
1               5                   10                  15


<210>  56
<211>  10
<212>  PRT
<213>  Artificial sequence

<220>
<223>  H-CDR3 D-F5

<400>  56

Ile Tyr Gly Asn Tyr Gly Arg Phe Ala Tyr
1               5                   10


<210>  57
<211>  16
<212>  PRT
<213>  Artificial sequence

<220>
<223>  L-CDR1 D-F5

<400>  57

Arg Ser Ser Gln Ser Ile Val His Ser Asn Gly Asn Thr Tyr Leu Glu
1               5                   10                  15


<210>  58
<211>  7
<212>  PRT
<213>  Artificial sequence

<220>
<223>  L-CDR2 D-F5

<400>  58

Lys Val Ser Asn Arg Leu Ser
1               5


<210>  59
<211>  5
<212>  PRT
<213>  Artificial sequence

<220>
<223>  H-CDR1 D-D6

<400>  59

Ser Asp Tyr Ala Trp
1               5

<210>  60
<211>  16
<212>  PRT
<213>  Artificial sequence

<220>
<223>  H-CDR2 D-D6

<400>  60

Tyr Ile Ser Tyr Ser Gly Arg Thr Ser Tyr Asn Pro Ser Leu Lys Ser
1               5                   10                  15


<210>  61
<211>  11
<212>  PRT
<213>  Artificial sequence

<220>
<223>  H-CDR3 D-D6

<400>  61

Leu Thr Thr Gly Val Gly Tyr Ala Met Asp Tyr
1               5                   10


<210>  62
<211>  16
<212>  PRT
<213>  Artificial sequence

<220>
<223>  L-CDR1 D-D6

<400>  62

Arg Ser Ser Gln Ser Leu Val His Ser Asn Gly Asn Thr Tyr Leu His
1               5                   10                  15


<210>  63
<211>  7
<212>  PRT
<213>  Artificial sequence

<220>
<223>  L-CDR2 D-D6

<400>  63

Lys Val Ser Asn Arg Phe Ser
1               5


<210>  64
<211>  3
<212>  PRT
<213>  Artificial sequence

<220>

<223>  H-CDR1 D-F5

<400>  64

Gly Phe Ser
1



<210>  65
<211>  3
<212>  PRT
<213>  Artificial sequence

<220>
<223>  H-CDR1 D-D6

<400>  65

Gly Tyr Ser
1



<210>  66
<211>  8
<212>  PRT
<213>  Artificial sequence

<220>
<223>  Amino acids 353-360 of human CK8

<400>  66

Leu Ser Glu Leu Glu Ala Ala Leu
1               5

## Description

### FIELD OF THE INVENTION

The present invention relates to the field of therapies using antibodies, or antibodies and chemotherapeutic agents. In particular, the invention relates to the use of anti-CK8 antibodies in the treatment of CK8 positive solid tumours. The invention relates to the use of an anti-CK8 antibody for K-Ras mutated solid tumours expressing CK8. The invention also relates to the use of anti-CK8 antibodies in a combination therapy with K-Ras wild-type (no K-Ras mutation). Combination therapy may be with an antibody directed against another target and/or with a chemotherapeutic agent in the treatment of CK8 positive solid tumours, with or without K-Ras mutation. The invention also relates to novel anti-CK8 antibodies having internalising property to deliver cytotoxic agent coupled to said antibody, in particular under the form of Antibody Drug Conjugate (ADC), for the treatment of CK8 positive solid tumours.

### BACKGROUND OF THE INVENTION

Surgery, chemotherapy, hormonal therapy and/or radiotherapy are the general therapeutic approaches to fight cancer. In the last decades, the use of biological therapy or immunotherapy has also been adopted. Still, many tumours respond only partially to the existing therapies and cases of resistance also occur. Therefore, there is a strong need for alternative cancer treatments.

The development of genome-wide association studies has enabled the discovery of genetic patterns, called biomarkers, associated with cancer, either participating to the oncogenesis or predictive of the therapeutic outcome.

Biomarkers are often classified as prognostic, pharmacodynamics or predictive biomarkers. Prognostic biomarkers help foreseeing the efficacy of a therapy and sometimes dictate if further therapy is sought. Pharmacodynamics biomarkers measure how effective is a drug against a disease. Predictive biomarkers anticipate the efficacy of a given treatment in terms of safety and/or efficacy. The number of predictive biomarkers routinely used in oncology is still pretty limited.

The best example is the HER2 overexpression in breast tumour, which predicts the efficacy of the anti HER2 MAb, trastuzumab. Other examples are K-RAS, the mutation of which predicts the inefficacy of cetuximab (anti EGFR MAb), BRC-ABL gene translocation for responsiveness to imatinib, BRAM mutation V600E to predict vemurafenib efficacy in melanoma and ALK rearrangement to predict crizotinib efficacy in non-small cell lung cancer.

Extracellular membrane-bound cytokeratin 8 (CK8) or portion of cytokeratin 8 has been detected in cells of several tumours, such as breast cancer (Godfroid et al, 1991, Journal of Cell Science. 99:595-607), cancers of the upper digestive tract (Gires et al, 2005, Biochemical and Biophysical Research Communications, 328: 1154-1162), colorectal cancer (WO2010/136536), head and neck cancer (Gires et al, 2006, Biochemical and Biophysical Research Communications 343: 252-259) and non-small cell lung cancer (Gharib et al, 2002, Neoplasia, 5:440-448).

Targeting externalized CK8 (eCK8) has been suggested as novel therapeutic approach for colorectal cancers (CRC) and other types of tumours. In fact, the preferential expression of CK8 on the outer membrane of tumour but not normal cells make of CK8 a promising target for anti-cancer therapy. WO 2016/020553 discloses anti-CK8 monoclonal antibodies (MAb) that are useful in the treatment of cancers expressing CK8, for example colorectal cancers.

Colorectal cancer (CRC) is the fourth leading cause of cancer death worldwide. CRC incidence was estimated at 1.4 million cases in 2012, with a mortality of 700 000 subjects. 50% of patients develop metastasis and the 5-year survival rate for these patients is of 10-20%. In developed countries, where more than 20% of CRC patients are diagnosed, a slow decline in incidence and mortality was recently observed. Instead, a sharp increase in CRC incidence and mortality was observed in countries that undergo economic growth in Europe, Asia and South America. Worldwide, the CRC burden is estimated to increase by 60% and up to 2.2 million cases in 2030, causing 1.1 million deaths.

In the past decade, the use of monoclonal antibodies targeting epidermal growth factor receptor (EGFR) have significantly improved the outcome in patients suffering of CRC. EGFR is overexpressed in a variety of tumours, including in 60%-80% of CRCs, and is directly implicated in disease initiation and progression, resistance to therapy and poor prognosis (Cunningham et al, 2004. N Engl J Med. 351:337-345.). Cetuximab, a chimeric IgG1 monoclonal antibody, binds EGFR in its inactive form. This prevents the ligand-induced signalling cascade downstream of EGFR (Li et al, 2005, Cancer Cell; 7:301-11), which occurs through the RAS-RAF-MAPK and PI3K- AKT-mTOR signalling cascades. By blocking EGFR signalling, cetuximab inhibits the EGFR-mediated proliferation, invasion and migration of the tumour (Ciardiello et al., 2008, N. Engl. J. Med. 358:1160-74).

A randomized clinical trial provided clear evidence of the efficacy of cetuximab only in patients with wild-type K-Ras gene. For this, screening for K-Ras mutations has been mandated by regulatory authorities for the selection of patients to be treated with cetuximab (Van Cutsem et al, 2009, N. Eng. J. Med.; 360:1408-1417). K-Ras encodes a small G protein that links ligand-dependent receptor activation to intracellular pathways of the EGFR signalling cascade.

Mutation at key sites within the gene, commonly at codons 12 and 13, causes constitutive activation of K-Ras-associated signalling (mutation is said to be an activating mutation in K-Ras). K-Ras mutation is observed in 9-30% of human cancers (Cox et al, 2014, Nat Rev Drug Discov. 13(11):828-51) and in 40% of CRC (Karapetis et al, 2008,. N Engl J Med. 359:1757-1765). Therefore there is a great medical need for the treatment of patients of patients suffering of metastatic CRC bearing activating mutation in K-Ras.

There is also still a strong need of a combinatory treatment capable of inducing tumour regression and/or remission in patients suffering of CK8 related tumours such as colorectal cancer (CRC), pancreas cancer (PC), Head & Neck cancer (H&N).

### SUMMARY OF THE INVENTION

The present invention relates to the use of an anti-CK8 antibody alone or in combi therapy (with another antibody and/or chemotherapeutic agent) to (1) treat solid tumours expressing CK8 having wild-type K-Ras (not mutated as disclosed herein) and (2) treat solid tumours expressing CK8 having K-Ras mutation as disclosed herein. The invention also relates to the use of anti-CK8 antibodies having internalising property, allowing to deliver cytotoxic agent coupled to antibody for the treatment of CK8 positive solid tumours, having wild-type K-Ras (not mutated as disclosed herein) or having K-Ras mutation as disclosed herein.

It has been found that tumours expressing CK8 and having K-Ras mutation are particularly sensitive to anti-CK8 antibody treatment by controlling the tumour growth. For instance, it has been found that the humanized monoclonal antibody HzMR022 (D-A10) inhibited the tumour growth having a K-Ras mutation (bearing activating mutation in K-Ras). The present invention thus relates to an anti-CK8 antibody such as HzMR022 (D-A10) for use in treating solid tumours, expressing CK8 and having K-Ras mutation.

The invention relates also to monoclonal antibody anti-CK8 combination therapy with chemotherapeutic drugs such as cisplatin for treating solid tumours expressing CK8 having a K-Ras mutation, particularly as disclosed herein, or having no K-Ras mutation in particular to inhibit or reduce the tumour growth.

The invention relates also the use of internalizing monoclonal antibody anti-CK8 to deliver cytotoxic agent to inhibit the tumour growth of CK8 positive solid tumours.

In an embodiment, the antibody, or antibody fragment thereof, specifically binding the peptide having the amino acid sequence of SEQ ID NO: 66. The antibody may comprise a heavy chain comprising the following three CDRs, respectively CDR-H1, CDR-H2 and CDR-H3, wherein CDR-H1 comprises the sequence SEQ ID NO: 8; CDR-H2 comprises the sequence SEQ ID NO: 9; and CDR-H3 comprises the sequence SEQ ID NO: 10. The antibody may comprise a light chain comprising the following three CDRs, respectively CDR-L1, CDR-L2 and CDR-L3, wherein CDR-L1 comprises the sequence SEQ ID NO: 5; CDR-L2 comprises the sequence SEQ ID NO: 6; and CDR-L3 comprises the sequence SEQ ID NO: 7. The antibody preferably comprises these heavy and light chains. As constant regions, the antibody may comprise the Heavy constant domain of SEQ ID NO: 18 and/or the Light constant region (kappa) of SEQ ID NO: 20

In an embodiment, the antibody, or antibody fragment thereof, is a humanized antibody, or antibody fragment thereof, specifically binding the peptide having the amino acid sequence of SEQ ID NO: 66. The humanized antibody may comprise a heavy chain comprising the following three CDRs, respectively CDR-H1', CDR-H2' and CDR-H3', wherein CDR-H1' comprises the sequence SEQ ID NO: 11; CDR-H2' comprises the sequence SEQ ID NO: 12; and CDR-H3' comprises the sequence SEQ ID NO: 10. The humanized antibody may comprise a light chain comprising the following three CDRs, respectively CDR-L1', CDR-L2' and CDR-L3', wherein CDR-L1' comprises the sequence SEQ ID NO: 13; CDR-L2' comprises the sequence SEQ ID NO: 6; and CDR-L3' comprises the sequence SEQ ID NO: 7. The antibody preferably comprises these heavy and light chains. As constant regions, the antibody may comprise the Heavy constant domain of SEQ ID NO: 18 and/or the Light constant region (kappa) of SEQ ID NO: 20.

In an embodiment, the antibody may comprise a heavy chain comprising the following three CDRs, respectively CDR-H1, CDR-H2 and CDR-H3, wherein CDR-H1 comprises the sequence SEQ ID NO: 43; CDR-H2 comprises the sequence SEQ ID NO: 44; and CDR-H3 comprises the sequence SEQ ID NO: 45. The antibody may comprise a light chain comprising the following three CDRs, respectively CDR-L1, CDR-L2 and CDR-L3, wherein CDR-L1 comprises the sequence SEQ ID NO: 46; CDR-L2 comprises the sequence SEQ ID NO: 47; and CDR-L3 comprises the sequence SEQ ID NO: 48. The antibody preferably comprises these heavy and light chains. As constant regions, the antibody may comprise the Heavy constant domain of SEQ ID NO: 18 and/or the Light constant region (kappa) of SEQ ID NO: 20..

The invention also relates to this mR022 (D-F5) antibody and a pharmaceutical composition comprising it and a pharmaceutically acceptable vehicle or excipient. This monoclonal anti-CK8 antibody have the VH sequence SEQ ID NO: 42 and the VL sequence SEQ ID NO: 40. As constant regions, the antibody may comprise the Heavy constant domain of SEQ ID NO: 18 and/or the Light constant region (kappa) of SEQ ID NO: 20.

In an embodiment, the antibody may comprise a heavy chain comprising the following three CDRs, respectively CDR-H1, CDR-H2 and CDR-H3, wherein CDR-H1 comprises the sequence SEQ ID NO: 49; CDR-H2 comprises the sequence SEQ ID NO: 50; and CDR-H3 comprises the sequence SEQ ID NO: 51. The antibody may comprise a light chain comprising the following three CDRs, respectively CDR-L1, CDR-L2 and CDR-L3, wherein CDR-L1 comprises the sequence SEQ ID NO: 52; CDR-L2 comprises the sequence SEQ ID NO: 47; and CDR-L3 comprises the sequence SEQ ID NO: 53. The antibody preferably comprises these heavy and light chains. As constant regions, the antibody may comprise the Heavy constant domain of SEQ ID NO: 18 and/or the Light constant region (kappa) of SEQ ID NO: 20.

The invention also relates to this mR022 (D-D6) antibody and a pharmaceutical composition comprising it and a pharmaceutically acceptable vehicle or excipient. This monoclonal anti-CK8 antibody have the VH sequence SEQ ID NO: 38 and the VL sequence SEQ ID NO: 36. As constant regions, the antibody may comprise the Heavy constant domain of SEQ ID NO: 18 and/or the Light constant region (kappa) of SEQ ID NO: 20.

These antibodies are defined by their CDRs of the VH and VL. However, their specific target is also disclosed. The person skilled in the art may thus appreciate that variations of some amino acids in the CDRs may be acceptable while keeping the affinity and functionality of the VH and VL sequences. As a result, the invention encompasses those variations of amino acid CDRs sequences. In particular, sequences with at least 80%, preferably 85%, 90%, 95% and 98%, identity after optimal alignment with sequence may be acceptable and determinable by routine experimentation. Also, the CDRs may be defined as disclosed herein using Kabat or Common numbering system.

The invention thus relates to a pharmaceutical composition comprising a monoclonal antibody as disclosed herein, and a pharmaceutically acceptable vehicle or carrier. In an embodiment, the composition comprises an antibody having the CDRs sequences of mR022 (D-F5) or of mR022 (D-D6), or one of these antibodies themselves, as provided herein.

Thus the present invention particularly relates to an anti-CK8 antibody, such as one disclosed herein, or a pharmaceutical composition comprising this antibody and a pharmaceutically acceptable vehicle, for use in treating solid tumours, expressing CK8 and having a K-Ras mutation (bearing activating mutation in K-Ras), such as colorectal (CRC), Head & Neck (HAN), Glioblastoma (GBM), Urinary (U), Prostate (PC), Breast (B), Lung (L), Pancreatic (PRC) or Renal (R) expressing CK8 and having a K-Ras mutation, in a patient in need thereof. The invention also relates to the use of such an antibody for the manufacture of a pharmaceutical composition for treating these tumours.

In some embodiments, the treated tumour has a K-Ras mutation selected from G13D, G12V, G12D, G61H, G12V or A146T mutations (see K-Ras sequence in Cox et al, 2014, Nat Rev Drug Discov. 13(11):828-51; (Karapetis et al, 2008,. N Engl J Med. 359:1757-1765). By having a K-Ras mutation it is meant that the CK8+ tumour cells also have at least one mutation in K-Ras, especially at least one of the listed mutations or any other K-Ras mutation that may render the CK8 positive tumour sensitive to anti-CK8 antibody therapy. Based on the present disclosure of the K-Ras status with respect to susceptibility to anti-CK8 antibody therapy, and method provided in the Example part, the person skilled in the art is able to determine K-Ras mutations that may qualify the tumour for such therapy in accordance with this aspect of the present invention.

The present invention also relates to personalized medicine, wherein the patient is tested for CK8 expression and/or K-Ras mutation. The patient may be tested for a K-Ras mutation particularly of the group listed above. The patient is selected for treatment with an anti-CK8 antibody if its tumour is tested positive for CK8 expression and/or K-Ras mutation, especially a K-Ras mutation as listed or provided herein, preferably positive for both CK8 and K-Ras mutation. Once selected, the patient may be treated with the anti-CK8 antibody, especially one of the herein-disclosed antibodies.

The present invention thus relates to an anti-cancer treatment comprising administering to a patient in need thereof an effective amount an anti-CK8 antibody for treating solid tumours expressing CK8 and having a K-Ras mutation, more particularly for colorectal (CRC), Head & Neck (HAN), Glioblastoma (GBM), Urinary (U), Prostate (PC), Breast (B), Lung (L), Pancreatic (PRC) or Renal (R). In an embodiment, the patient is tested for CK8 expression and/or K-Ras mutation before treatment, and is treated with an anti-CK8 antibody if its tumour is tested positive for CK8 expression and/or K-Ras mutation. In an embodiment, the patient tested positive for CK8 expression and K-Ras mutation according to the invention is treated with at least one of the monoclonal anti-CK8 antibodies disclosed herein. The testing may comprise testing for one of the K-Ras mutations G13D, G12V, G12D, G61H, G12V or A146T.

The invention thus relates also to monoclonal antibody anti-CK8 combination therapy with chemotherapeutic drugs such as cisplatin for treating solid tumours expressing CK8 and having no K-Ras mutation or having a K-Ras mutation, particularly as disclosed herein, in particular to inhibit or reduce the tumour growth.

The present invention especially relates to an anti-CK8 monoclonal antibody, such as one disclosed herein, or a pharmaceutical composition comprising this antibody and a pharmaceutically acceptable vehicle, for use in treating solid tumours expressing CK8, more particularly for colorectal (CRC), Head & Neck (HAN), Glioblastoma (GBM), Urinary (U), Prostate (PC), Breast (B), Lung (L), Pancreatic (PRC) or Renal (R) expressing CK8, in a patient in need thereof as part of a combination therapy with a chemotherapeutic agent. In an embodiment, the tumour has a K-Ras mutation (bearing activating mutation in K-Ras), especially one of the above-listed mutations. The invention also relates to the use of such an antibody for the manufacture of a pharmaceutical composition for treating these tumours in combination with such a chemotherapeutic agent.

In an embodiment, this anti-CK8 antibody is for use in treating HAN cancer expressing CK8, and no K-Ras mutation. More particularly this antibody is for use in combination therapy with another agent such as a chemotherapeutic drug. Say chemotherapeutic drug may be cisplatin. The invention also relates to the use of such an antibody for the CK8 positive tumour treatment with no K-Ras mutation such as colorectal (CRC), Head & Neck (HAN), Glioblastoma (GBM), Urinary (U), Prostate (PC), Breast (B), Lung (L), Pancreatic (PRC) or Renal (R).

The present invention also relates to a combined therapy against tumours whose cells express CK8 protein, and possibly have no K-Ras mutation, in particular a H&N cancer, using an anti-CK8 antibody according to the present invention and a chemotherapeutic drug as disclosed herein, in particular cisplatin. The anti-CK8 antibody and the chemotherapeutic drug are for use in treating tumours whose cells express CK8 protein, and having no K-Ras mutation, in particular a HAN cancer. The method of use or the anti-cancer treatment includes administering to a patient in need thereof a sufficient amount of an anti-CK8 antibody according to the present invention and a sufficient amount of a chemotherapeutic drug such as cisplatin. Antibody and drug may be administered in a simultaneous, separate or sequential way.

The present invention also relates to a kit or pharmaceutical composition comprising a first composition comprising an anti-CK8 antibody according to the present invention and a suitable pharmaceutical carrier and a second composition comprising a chemotherapeutic drug as disclosed herein, in particular cisplatin, and a suitable pharmaceutical carrier, in particular for its use in treating tumours expressing CK8, and possibly no K-Ras mutation, in particular a HAN cancer. The first and the second compositions may be for simultaneous, separate or sequential administration to a patient in need thereof.

The invention thus relates also to the use of internalizing anti-CK8 monoclonal antibody, especially mR022 (D-A10, D-D6 or D-F5) for use in treating tumours expressing CK8, and possibly have a K-Ras mutation and possibly no K-Ras mutation in particular CRC.

The present invention particularly relates to monoclonal antibody mR022 D-A10, D-D6 or D-F5) or a monoclonal antibody comprising the CDRs of this mR022D-A10, D-D6 or D-F5), or a pharmaceutical composition comprising this antibody and a pharmaceutically acceptable vehicle, for use in treating tumours expressing CK8, and possibly have a K-Ras mutation or no K-Ras mutation, in particular colorectal (CRC), Head & Neck (HAN), Glioblastoma (GBM), Urinary (U), Prostate (PC), Breast (B), Lung (L), Pancreatic (PRC) or Renal (R). In an embodiment internalizing monoclonal antibodies anti-CK8 such as mR022 (D-A10, D-D6, D-F5) are used to deliver cytotoxic agent to inhibit the tumour growth of CK8 positive solid tumours.

The present invention also relates to monoclonal antibody mR022 (DA10) or HzR022 'DA10), or a monoclonal antibody comprising the CDRs thereof, or a pharmaceutical composition comprising this antibody and a pharmaceutically acceptable vehicle, for use in treating tumours expressing CK8, and possibly have a K-Ras mutation or no K-Ras mutation, in particular colorectal (CRC), Head & Neck (HAN), Glioblastoma (GBM), Urinary (U), Prostate (PC), Breast (B), Lung (L), Pancreatic (PRC) or Renal (R).

It also relates to a method of use or anti-cancer treatment including administering to a patient in need thereof an effective amount of this anti-CK8 antibody according to the present invention to a patient in need thereof, especially having colorectal (CRC), Head & Neck (HAN), Glioblastoma (GBM), Urinary (U), Prostate (PC), Breast (B), Lung (L), Pancreatic (PRC) or Renal (R). Use of a humanized version of said antibody is preferred. In an embodiment this internalizing monoclonal antibody anti-CK8 such as mR022 (D-D6) is used to deliver cytotoxic agent to inhibit the tumour growth of CK8 positive solid tumours.

The present invention provides several monoclonal antibodies defined by their CDRs sequences as disclosed herein. The present invention also relates to a pharmaceutical composition comprising such an antibody or an effective antibody fragment thereof, and a suitable pharmaceutical vehicle or carrier. These compositions are in particular for use in the treatment of a cancer, and/or as a medicament to induce apoptosis of a tumour cell, in particular for use in the treatment of tumours whose cells express CK8 protein, and having or not having a K-Ras mutation, as disclosed herein.

The present invention also relates to a kit or pharmaceutical composition comprising a first composition comprising an anti-CK8 antibody according to the present invention and a suitable pharmaceutical carrier and a second composition comprising another antibody (antibody directed against another target than CK8) or chemotherapeutic drug as disclosed herein and a suitable pharmaceutical carrier. This kit or composition is in particular for use in treating tumours expressing CK8, in particular for use in treating colorectal (CRC), Head & Neck (HAN), Glioblastoma (GBM), Urinary (U), Prostate (PC), Breast (B), Lung (L), Pancreatic (PRC) or Renal (R) expressing CK8, having or not having a K-Ras mutation according to the invention. It may also be used to treat glioblastoma or prostate cancer. The first and the second composition may be for simultaneous, separate or sequential administration to a patient in need thereof.

The present invention also relates to methods for treating tumours expressing CK8, in particular for treating colorectal (CRC), Head & Neck (HAN), Glioblastoma (GBM), Urinary (U), Prostate (PC), Breast (B), Lung (L), Pancreatic (PRC) or Renal (R) expressing CK8, having or not having a K-Ras mutation according to the invention, wherein an antibody according to the invention, or a pharmaceutical composition containing it as disclosed herein is administered in effective amount to the patient. In an embodiment, the patient is also administered with another antibody against another target on the same tumour cells and/or a chemotherapeutic drug, for example cisplatin.

The invention also relates to the use of such antibodies for the manufacture of a pharmaceutical composition for treating these tumours.

### DETAILED DESCRIPTION OF THE INVENTION

### Anti-CK8 antibody

The CDR sequences are defined in accordance with IMGT^{®}, Kabat^{®} or the common numbering system which retains sequences common to IMGT^{®} and Kabat^{®} (see Examples section). The CDRs of the antibodies of the present invention, in particular of the mR022 (D-A10) Mab, are presented in this Table :

| | SEQ ID NO: | IMGT sequence | SEQ ID NO: | Kabat sequence | SEQ ID NO: | Sequence (Common numbering system) |
|---|---|---|---|---|---|---|
| VL mD-A10 | | | | | | |
| CDR1 | 5 | KSLLYSNGNTY | 22 | RSSKSLLYSNGNTYL Y | 5 | KSLLYSNGNTY |
| CDR2 | 6 | YMS | 23 | YMSNLAS | 6 | YMS |
| CDR3 | 7 | MQSLEYPFT | 7 | MQSLEYPFT | 7 | MQSLEYPFT |

| VH mD-A10 | | | | | | |
|---|---|---|---|---|---|---|
| CDR1 | 8 | GFTFSGFW | 24 | GFWMS | 25 | GFW |
| CDR2 | 9 | INSDGSAI | 26 | DINSDGSAIKYAPSIK D | 9 | INSDGSAI |
| CDR3 | 10 | IAHYSGGGFAY | 27 | HYSGGGFAY | 27 | HYSGGGFAY |

The CDR sequences of humanized antibodies of the present invention, in particular of HzR022 (D-A10) Mab, are summarized in this Table :

| | SEQ ID NO: | Sequence IMGT | SEQ ID NO: | Sequence Kabat | SEQ ID NO: | Sequence (Common numbering system) |
|---|---|---|---|---|---|---|
| VH Hz D-A10 | | | | | | |
| CDR1 | 11 | GFTFSSYW | 28 | SYWMS | 29 | SYW |
| CDR2 | 12 | INSDGSST | 30 | DINSDGSSTKYAPSIK D | 12 | INSDGSST |
| CDR3 | 10 | IAHYSGGGFAY | 31 | HYSGGGFAY | 32 | HYSGGGFAY |

| VL Hz D-A10 | | | | | | |
|---|---|---|---|---|---|---|
| CDR1 | 13 | KSLLYSNGYNY | 33 | RSSKSLLYSNGYNYL Y | 13 | KSLLYSNGYNY |
| CDR2 | 6 | YMS | 34 | YMSNLAS | 6 | YMS |
| CDR3 | 7 | MQSLEYPFT | 7 | MQSLEYPFT | 7 | MQSLEYPFT |

The CDR sequences of antibodies of the present invention, in particular of mR022 (D-F5) Mab, are summarized in this Table :

| | SEQ ID NO: | Sequence IMGT® | SEQ ID NO: | Sequence Kabat® | SEQ ID NO: | Sequence (Common numbering system) |
|---|---|---|---|---|---|---|
| VH mR022 (D-F5) | | | | | | |
| CDR1 | 43 | GFSLTSYG | 54 | SYGVH | 64 | GFS |
| CDR2 | 44 | IWAGGST | 55 | VIWAGGSTNYNSALM S | 44 | IWAGGST |
| CDR3 | 45 | ARIYGNYGRFAY | 56 | IYGNYGRFAY | 56 | IYGNYGRFAY |

| VL mR022 (D-F5) | | | | | | |
|---|---|---|---|---|---|---|
| CDR1 | 46 | QSIVHSNGNTY | 57 | RSSQSIVHSNGNTYL E | 46 | QSIVHSNGNTY |
| CDR2 | 47 | KVS | 58 | KVSNRLS | 47 | KVS |
| CDR3 | 48 | FQGSLVPLT | 48 | FQGSLVPLT | 48 | FQGSLVPLT |

The CDR sequences of antibodies of the present invention, in particular of mR022 (D-D6) Mab, are summarized in this Table :

| | SEQ ID NO: | Sequence IMGT® | SEQ ID NO: | Sequence Kabat® | SEQ ID NO: | Sequence (Common numbering system) |
|---|---|---|---|---|---|---|
| VH mR022 (D-D6) | | | | | | |
| CDR1 | 49 | GYSITSDY | 59 | SDYAW | 65 | GYS |
| CDR2 | 50 | ISYSGRT | 60 | YISYSGRTSYNPSLK S | 50 | ISYSGRT |
| CDR3 | 51 | APLTTGVGYAM DY | 61 | LTTGVGYAMDY | 66 | LTTGVGYAMDY |

| VL mR022 (D-D6) | | | | | | |
|---|---|---|---|---|---|---|
| CDR1 | 52 | QSLVHSNGNTY | 62 | RSSQSLVHSNGNTYL H | 52 | QSLVHSNGNTY |
| CDR2 | 47 | KVS | 63 | KVSNRFS | 47 | KVS |
| CDR3 | 53 | SQSTHVPFT | 53 | SQSTHVPFT | 53 | SQSTHVPFT |

By definition, these CDRs include variant CDRs, by deletion, substitution or addition of one or more amino acid(s), which variant retains the specificity of the original CDR, of the variable region or the specificity of the antibody. The common numbering system provides for a CDR definition having the shortest amino acid sequences or the minimal CDR definition.

According to a feature, the anti-CK8 antibodies the invention may be, or may have been, produced in mammal cells. The mammal cell may be a wild-type cell. It may be a rodent cell, in particular a CHO cell. The rodent cell may be wild-type, such as in particular a wild-type CHO. The antibodies have a glycosylation profile resulting from their production in that cell. Wild-type is used in its usual meaning, say is relates to the phenotype of the typical form of a species as it occurs in nature.

In an embodiment, the Heavy chain of the antibodies of the invention comprise the variable VH domain as disclosed herein, and a constant domain comprising CH1, hinge, CH2 and CH3. This constant domain is preferably as depicted on SEQ ID NO: 18, or as encoded by the nucleotide sequence on SEQ ID NO: 19.

In an embodiment, the Light chain of the antibodies of the invention comprises the variable VL domain as disclosed herein, and a constant CI domain. This constant domain is preferably a kappa chain, especially as depicted on SEQ ID NO: 20, or as encoded by the nucleotide sequence on SEQ ID NO: 21.

In an embodiment, the antibodies of the invention comprise these Heavy and Light chains.

### Methods for producing the antibodies are known from the person skilled in the art.

The antibodies of the present invention are preferably monoclonal antibodies. Said monoclonal antibodies may be murine, chimeric or humanized, bispecific, multivalent or ADC antibodies, They may be obtained by standard methods well-known to the person skilled in the art.

For producing the anti-CK8 antibody of the invention, the mammal cells, preferably rodent cells such as CHO cells, preferably wild-type cells (e.g. wild-type CHO cells) may be transfected with one or several expression vectors. Preferably, the cells may be co-transfected with an expression vector comprising a nucleotide sequence coding for Light chain and with an expression vector comprising a nucleotide sequence coding for Heavy chain. For the production of antibodies useful in the invention, the person skilled in the art may refer to WO2016/020553, which is incorporated herein by reference.

Monoclonal antibodies, in particular of murine origin, can be prepared according to the techniques described in the manual *Antibodies* (Harlow and Lane, Antibodies: A Laboratory Manual, Cold Spring Harbor Laboratory, Cold Spring Harbor NY, pp. 726, 1988) or prepared from hybridomas. Such techniques are well known from the person skilled in the art.

Alternatively, the monoclonal antibodies of the present invention can be obtained, for example, from cells of an animal immunized with a human CK8 protein fragment as disclosed in WO2016/020553. The monoclonal antibodies according to the invention can, for example, be purified on an affinity column on which has been immobilized before a suitable human CK8 fragment. Other purification techniques are well known, for example, purification on an affinity column.

Chimeric antibodies can be prepared using genetic recombination techniques. For example, the chimeric antibody can be produced by cloning a recombinant DNA having a promoter and a sequence encoding the variable region of a non-human, in particular murine monoclonal antibody and a sequence encoding the constant region of the human antibody. A chimeric antibody of the invention encoded by such a recombinant gene will be, for example, a mouse-human chimera, the specificity of this antibody being determined by the variable region derived from the murine DNA and its isotype being determined by the constant region derived from the human DNA. Methods for producing chimeric antibodies are extensively described in the literature.

Humanized antibodies can be prepared by techniques known to a person skilled in the art (such as, for example, those described in Singer et al., J. Immun., 150:2844-2857, 1992; Mountain et al., Biotechnol. Genet. Eng. Rev., 10:1-142, 1992; and Bebbington et al., Bio/Technology, 10:169-175, 1992). Other humanization techniques, also known to a person skilled in the art, such as, for example, EP 0 682 040, EP 0 939 127, EP 0 566 647, US 6,180,370, US 5,585,089, US 5,693,761., US 6,054,297, US 5,886,152 and US 5,877,293.

To minimize anti-V region responses, monoclonal humanized antibodies may be prepared by grafting onto the human templates only the specificity-determining residues (SDRs), i.e. the residues that are essential for the surface complementarity of the Mab and its antigen. To that end, murine antibodies may be humanized by grafting their complementarity determining regions (CDRs) onto the variable light (VL) and variable heavy (VH) frameworks of human immunoglobulin molecules, while retaining those murine framework residues deemed essential for the integrity of the antigen-combining site. However, as the xenogeneic CDRs of the humanized antibodies may evoke anti-idiotypic (anti-Id) response in patients, the humanized antibodies of the invention or fragments thereof can be prepared by techniques minimizing the anti-Id response. Examples of these techniques include the grafting, onto the human frameworks, of only the specificity determining residues (SDRs), i.e. the CDR residues that are most crucial in the antibody-ligand interaction. The SDRs are identified through the help of the database of the three-dimensional structures of the antigen-antibody complexes of known structures or by mutational analysis of the antibody-combining site. An alternative approach to humanization, which involves retention of more CDR residues, is based on grafting of the 'abbreviated' CDRs, i.e. the stretches of CDR residues that include all the SDRs.

Functional antibody fragments can be obtained from the antibodies herein described by enzymatic digestion, for example by means of pepsin or papain, and/or by cleavage of disulfide bridges by chemical reduction. Alternatively, the antibody fragments of the present invention can be obtained by gene recombination techniques or by peptide synthesis. These methods are well-known to the person skilled in the art.

The antibodies or antibody fragments of the present invention are preferably antibodies or antibody fragments selected from murine, chimeric, humanized bivalent, multivalent or ADC antibodies, preferably having an optimized sequence.

The antibodies of the present invention, or antibody fragments thereof, may comprise the VH and VL sequences as disclosed herein or their whole Heavy and Light sequences as disclosed herein, or their variant sequences with at least 80%, preferably 85%, 90%, 95% and 98%, identity after optimal alignment.

### Combination therapy

As used herein the term "combination" is used in its broadest sense and means that a subject is treated with at least two therapeutic regimens or pharmaceutical compositions or drugs. As used herein, the term "drugs" may encompass antibody and chemotherapeutic agent, as appropriate depending on the context.

Thus, "combination antibody therapy" for treating CK8 positive tumours is intended to mean a subject is treated with at least two monoclonal antibodies, pharmaceutical compositions containing each a monoclonal antibody, or antibody regimens, more particularly, with at least one anti-CK8 antibody according to the invention and with at least one another antibody directed against another target or receptor on the tumour cells. The timing of administration of the different antibody/compositions/regimens can be varied so long as the beneficial effects of the combination of these antibodies is achieved. Treatment with the two antibodies can be at the same time (e.g. simultaneously or concurrently), or at different times (e.g. consecutively or sequentially), or a combination thereof. "Combination therapy" may also be achieved using a bispecific antibody targeting CK8 and the other target or receptor.

Also, "combination therapy" for treating CK8 positive tumours is intended to mean a subject is treated with at least two drug regimens, more particularly, with at least one chemotherapeutic agent, e.g. cisplatin, in combination with at least one anti-CK8 antibody according to the invention, or pharmaceutical composition containing the same. The timing of administration of the different regimens can be varied so long as the beneficial effects of the combination of these drugs is achieved. Treatment with a chemotherapeutic agent, e.g. cisplatin, in combination with an anti-CK8 antibody can be at the same time (e.g. simultaneously or concurrently), or at different times (e.g. consecutively or sequentially), or a combination thereof.

For the purposes of the present disclosure, administering at the same time (e.g., simultaneously) refers to administering the drugs together in same formulation or in separate formulations wherein the administration may be a few minutes to a few hours apart, but no more than one day. As used herein administering at different times (e.g., sequentially) refers to administering the drugs of the combination therapy a few hours to days, weeks and even months apart.

Therefore, in certain embodiments a subject undergoing combination therapy can receive both drugs at the same time (e.g., simultaneously) or at different times (e.g., sequentially, in either order, on the same day, or on different days), as long as the therapeutic effect of the combination of both drugs is caused in the subject undergoing therapy. In some embodiments, the combination of drugs will be given simultaneously for one dosing, but other dosing will include sequential administration, in either order, on the same day, or on different days. Where the two drugs are administered simultaneously, they can be administered as separate pharmaceutical compositions, each comprising either drug of the combination, or can be administered as a single pharmaceutical composition comprising both of these drugs.

Examples of agents that can be used in these combinations may be the so-called agents for targeted therapy. These agents interfere with tumour growth by impairing specific molecular mechanisms that participate to tumour initiation and/or progression. Agents for targeted therapy may be small molecules or antibodies and are commonly classified according to the molecular target. Examples of molecular targets of targeted therapy are proteins participating to cell signalling, apoptosis, gene transcription, DNA repair, cell cycle progression and/or checkpoint, angiogenesis, invasion and metastasis.

Targeting CK8 with the antibodies of the present invention in combination with existing chemotherapeutic treatments will be more effective in killing the tumour cells than chemotherapy alone.

Chemotherapeutic agents that can be used in the combination of the invention can be classified in groups according to their mode of action. A non-exhaustive list of chemotherapy classes follows hereafter: alkylating agents (e.g cyclophosphamide), anthracyclines (e.g. doxorubicin), cytoskeletal disruptors (e.g. paclitaxel), epothilones (e.g. ibxabepilone), histone deacetylase inhibitors (e.g. vorinostat), inhibitors of topoisomerase (e.g. irinotecan), kinase inhibitors (e.g. imatinib), nucleotide analogues and precursor analogues (e.g. azacytidine), peptide antibiotics (e.g. bleomycin), platinum-based agents (e.g. cisplatin), retinoid (e.g. tretinoin), vinca alkaloids and derivatives (e.g. cisplatin).

Other agents used for cancer therapy, commonly classified as agents for hormonal therapy, include hormones, inhibitors of hormone synthesis, hormone receptor antagonists.

In one approach, antibody treatment or regimen, including combination of at least two antibodies, may be added to a standard chemotherapy regimen, in treating a cancer patient.

For those combinations in which the antibody and additional anti-cancer agent(s) exert a synergistic effect against cancer cells, the dosage of the additional agent(s) may be reduced, compared to the standard dosage of the second agent when administered alone. The antibody may be co-administered with an amount of an anti-cancer drug (including antibody) that is effective in enhancing sensitivity of cancer cells.

In one method of the invention, the antibody targeting CK8 is administered to the patient simultaneously or at the same time with the administration of a chemotherapeutic agent. One alternative method comprises administering the chemotherapeutic agent prior to administering the antibody. Another alternative method comprises administering the antibody prior to administering the chemotherapeutic agent.

The method of the invention may provide for the inclusion in a therapeutic regimen involving the use of at least one other treatment method, such as irradiation, chemotherapy with small molecule or antibody. The method of the invention may directly include the administration of a sufficient amount of at least one additional antibody directed against another target and/or at least one chemotherapeutic drug (such as small molecule), for a simultaneous, separate or sequential administration with antibody(ies) of the invention, to a mammal, including man. This combination more generally is useful for cancers (in particular aggressive cancers) which do not respond well to treatment with the drug alone or the antibodies/antibody of the invention alone, and for which the combination leads to a synergistic effect.

The antibodies of the invention may be a monoclonal antibody, a chimeric antibody, a humanized antibody, a full human antibody, a bispecific antibody (e.g. against CK8 and another antigen), an association of at least two antibodies, a multivalent antibody composition, an antibody drug conjugate or an antibody fragment with one or two specificities at least. A "humanized antibody" or "chimeric humanized antibody" shall mean an antibody derived from a non-human antibody, typically a murine antibody, that retains or substantially retains the antigen-binding properties of the parental non-human antibody, but which is less immunogenic in humans.

### Therapeutic activity and treatment regimen

Tumour response can be assessed for changes in tumour morphology (e.g., overall tumour burden, tumour size, and the like) or disappearance of tumour using the usual techniques at the disposal of the clinicians and laboratories, such as screening techniques such as magnetic resonance imaging (MBS) scan, x-radiographic imaging, computed tomographic (CT) scan, CK8 positive tumours flow cytometry or CK8 positive tumours fluorescence-activated cell sorter (FACS) analysis, bioluminescent imaging, for example, luciferase imaging, bone scan imaging, and tumour biopsy sampling including bone marrow aspiration (BMA). The methods of the disclosure comprise using combination therapy which confers a positive therapeutic response to a subject in need of a treatment for CK8 positive tumours, in particular with K-Ras mutation according to the invention. A positive therapeutic response with respect to the combination treatment using an another antigen antibody and an anti-CK8 antibody (e.g. to treat CK8 positive tumours) or using an anti-CK8 antibody and a chemotherapeutic agent, such as cisplatin (e.g. to treat CK8 positive tumours) in particular with no K-Ras mutation according to the invention is intended to mean an improvement in the disease in association with the anti-tumour activity of these drugs, and/or an improvement in the symptoms associated with the disease. That is, an anti-proliferative effect, the prevention of further tumour growth, a reduction in tumour size, a reduction in the number of cancer cells, can be observed. Thus, for example, an improvement in the disease may be characterized as a complete response.

The term "Regression" means a reduction in the size of the tumour mass; a reduction in metastatic invasiveness of the tumour; a reduction in the rate of tumour growth; an increased patient survival rate; and/or an increase in observed clinical correlates of improved prognosis such as increased tumour infiltrating lymphocytes and decreased tumour vascularization; and the like. Regression may be regarded as a "partial response", say at least about a 50% decrease in all measurable tumour burden (e.g., the number of tumour cells present in the subject) in the absence of new lesions and persisting for at least one month.

The term "Remission" means that the tumour or the tumour cells are no longer detectable. Remission may be regarded as a "complete response", say an absence of clinically detectable disease with normalization of any previously abnormal radiographic studies. Such a response must persist for at least one month following treatment according to the methods of the disclosure.

A pharmaceutical composition or a combination of the invention can be used as a "therapeutic composition" to inhibit growth of mammalian, particularly human, cancer cells as a combination therapy, and/or in further combination with radiation therapy. An effective amount of a pharmaceutical composition is administered preferably to inhibit or reverse progression of cancers that are expressing CK8, or otherwise result in a statistically significant increase in remission, or progression-free survival (i.e., the length of time during and after treatment in which a patient is living with said targeted cancer, i.e. CK8 positive tumours or CK8 positive tumours having or not having K-Ras mutation, that does not get worse), or overall survival (also called "survival rate"; i.e., the percentage of people in a study or treatment group who are alive for a certain period of time after they were diagnosed with or treated for cancer) relative to treatment with a control.

The antibodies or compositions of the invention are administered at a therapeutically effective dose. The term "therapeutically effective dose," "therapeutically effective amount", or "effective amount" is intended to be an amount of the anti-CK8 antibody that brings about a positive therapeutic response with respect to treatment of a subject for a CK8 positive tumours and CK8 positive tumours having or not having K-Ras mutation according to the invention.

When administered in combination with an amount of another antibody (e.g. in treating CK8 positive tumours, possibly with K-Ras mutation according to the invention or no mutation) or the chemotherapeutic agent (e.g. in treating CK8 positive tumours, possibly with K-Ras mutation according to the invention), the term "therapeutically effective dose," "therapeutically effective amount," or "effective amount" is intended to be an amount of the anti-CK8 antibody that brings about a positive therapeutic response with respect to treatment of a subject for a CK8 positive tumours and CK8 positive tumours having or not having K-Ras mutation according to the invention.

In some embodiments, a therapeutically effective dose of the anti-CK8 antibody of the invention and/or the other antibody or chemotherapeutic agent is in the range from about 0.1 mg/kg to about 200 mg/kg, for example from about 1 mg/kg to up to about 100 mg/kg. In some embodiments, the dosage can be 1, 3, 5, 10, 15, 20, 25, or 30 mg/kg. The invention provides combination therapy or regimen with at least two different antibodies or at least one antibody and at least one chemotherapeutic agent. By result of this, the therapeutic effective dose of one drug (antibody or chemotherapeutic agent) required for a given therapeutic effect may be lower than if used alone, so the low dosage values (e.g. equal or less than 60 mg/kg) given may be sufficient amount, e.g. 1, 3, 5, 10, 15, 20, 25, or 30 mg/kg.

Such "therapeutically effective dose," "therapeutically effective amount," or "effective amount" can be routinely determined by those of skilled in the art. The amount of the compound actually administered will typically be determined by a physician, in the light of the relevant circumstances, including the condition to be treated, the chosen route of administration, the actual compound administered the age, weight, and response of the individual patient, the severity of the patient's symptoms, etc. It will also be appreciated by those of stalled in the art that the dosage may be dependent on the stability of the administered peptide.

The pharmaceutical compositions can be administered by injection, that is, intravenously, intramuscularly, intracutaneously, subcutaneously, intraduodenally or intraperitoneally. Other pharmaceutical delivery systems can also be employed, for example, liposomes.

An anti-CK8 monoclonal antibody (or composition containing it) of the present invention can be administered prior to and/or subsequent to (collectively, "sequential treatment"), and/or simultaneously with ("concurrent treatment") a specific second monoclonal antibody or a chemotherapeutic agent according to the present invention. Sequential treatment (such as pretreatment, post-treatment, or overlapping treatment) of the combination, also includes regimens in which the drugs are alternated, or wherein one component is administered long-term and the other(s) are administered intermittently. Components of the combination may be administered in the same or in separate compositions, and by the same or different routes of administration.

For any compound, the therapeutically effective dose can be estimated initially either in cell culture assays or in animal models such as mice, rats, rabbits, dogs, pigs, or monkeys. An animal model may also be used to determine the appropriate concentration range and route of administration. Such information can then be used to determine useful doses and routes for administration in humans. The exact dosage will be determined in light of factors related to the subject requiring treatment. Dosage and administration are adjusted to provide sufficient levels of the active compound or to maintain the desired effect. Factors that may be taken into account include the severity of the disease state, the general health of the subject, the age, weight, and gender of the subject, time and frequency of administration, drug combination(s), reaction sensitivities, and response to therapy. Long-acting pharmaceutical compositions may be administered every 3 to 4 days, every week, or biweekly depending on the half-life and clearance rate of the particular formulation. The frequency of dosing will depend upon the pharmacokinetic parameters of the molecule in the formulation used. Typically, a composition is administered until a dosage is reached that achieves the desired effect. The composition may therefore be administered as a single dose, or as multiple doses (at the same or different concentrations/dosages) over time, or as a continuous infusion. Further refinement of the appropriate dosage is routinely made. Appropriate dosages may be ascertained through use of appropriate dose-response data.

### Pharmaceutical compositions

A pharmaceutical composition comprises at least one monoclonal antibody according to the invention, and a pharmaceutically acceptable vehicle or carrier.

A pharmaceutical composition of the present invention may contain formulation materials for modifying, maintaining or preserving, for example, the pH, osmolarity, viscosity, clarity, color, isotonicity, odor, sterility, stability, rate of dissolution or release, adsorption, or penetration of the composition.

The primary vehicle or carrier in a pharmaceutical composition may be either aqueous or non-aqueous in nature. For example, a suitable vehicle or carrier may be water for injection or physiological saline, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which may further include sorbitol or a suitable substitute therefore. In one embodiment of the present invention, binding agent compositions may be prepared for storage by mixing the selected composition having the desired degree of purity with optional formulation agents (Remington's Pharmaceutical Sciences, supra) in the form of a lyophilized cake or an aqueous solution. Further, the binding agent product may be formulated as a lyophilizate using appropriate excipients such as sucrose.

The formulation components are present in concentrations that are acceptable to the site of administration. For example, buffers are used to maintain the composition at physiological pH or at slightly lower pH, typically within a pH range of from about 5 to about 8. A particularly suitable vehicle for parenteral administration is sterile distilled water in which a binding agent is formulated as a sterile, isotonic solution, properly preserved. Yet another preparation can involve the formulation of the desired molecule with an long-lasting agent that provide for the controlled or sustained release of the product which may then be delivered via a depot injection (such as injectable microspheres, bio-erodible particles, polymeric compounds (polylactic acid, polyglycolic acid), beads, or liposomes).

In another aspect, pharmaceutical formulations suitable for parenteral administration may be formulated in aqueous solutions, preferably in physiologically compatible buffers such as Hank's' solution, Ringer's solution, or physiologically buffered saline. Aqueous injection suspensions may contain substances that increase the viscosity of the suspension, such as sodium carboxymethyl cellulose, sorbitol, or dextran. Additional pharmaceutical compositions will be evident to those skilled in the art, including formulations involving binding agent molecules in sustained- or controlled-delivery formulations.

Techniques for formulating a variety of other sustained- or controlled-delivery means, such as liposome carriers, bio-erodible microparticles or porous beads and depot injections, are also known to those skilled in the art. The pharmaceutical composition to be used for *in vivo* administration typically must be sterile. This may be accomplished by filtration through sterile filtration membranes. Where the composition is lyophilized, sterilization using this method may be conducted either prior to or following lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (e.g., lyophilized) requiring reconstitution prior to administration.

### Monoclonal antibodies useful in the invention:

CDRs of these antibodies have been disclosed above in the above Tables.

### Variable regions of Murine monoclonal antibody D-A10 is referred to herein as "m-R022 (D-A10) Mab":

VL mD-A10 - Mab SEQ ID NO: 1: 
AACATTGTTATGACCCAGGCCGCACCCTCTGTACCTGTCACTCCTGGAGAGTCAGTA
TCCATCTCCTGCAGGTCTAGTAAGAGTCTTCTGTATAGTAATGGCAACACTTATTTGT
ATTGGTTCCTGCAGAGGCCAGGCCAGTCTCCTCAGCGCCTGATATATTATATGTCCA
ACCTTGCCTCAGGAGTCCCAGACAGGTTCAGTGGCAGAGGGTCAGGAACTGATTTC
ACACTGAGAATCAGTAGAGTGGAGGCTGAGGATGTGGGTGTTTATTACTGTATGCAA
AGTCTAGAATATCCTTTCACGTTCGGTGGAGGCACCAAGCTCGAGATCAAA.
VL mD-A10 - Mab SEQ ID NO: 2 : 
NIVMTQAAPSVPVTPGESVSISCRSSKSLLYSNGNTYLYWFLQRPGQSPQRLIYYMSNLA
SGVPDRFSGRGSGTDFTLRISRVEAEDVGVYYCMQSLEYPFTFGGGTKLEIK.
VH mD-A10 Mab - SEQ ID NO: 3: 
AAGTGCAGCTGTTGGAGACTGGAGGAGGCTTGGTGCAACCGGGGGGGTCACGGGG
ACTCTCTTGTGAAGGCTCAGGGTTTACTTTTAGTGGCTTCTGGATGAGCTGGGTTCG
ACAGACACCTGGGAAGACCCTGGAGTGGATTGGAGACATTAATTCTGATGGCAGTGC
AATAAAATACGCACCATCCATAAAGGATCGATTCACTATCTTCAGAGACAATGACAAG
AGCACCCTGTACCTGCAGATGAGCAATGTGCGATCTGAGGACACAGCCACGTATTTC
TGTATCGCCCATTACTCCGGTGGGGGGTTTGCTTACTGGGGTCAAGGAACCTCGGT
CACCGTCTCCTCA.
VH mD-A10 Mab - SEQ ID NO: 4 :

VQLLETGGGLVQPGGSRGLSCEGSGFTFSGFWMSWVRQTPGKTLEWIGDINSDGSAIK
YAPSIKDRFTIFRDNDKSTLYLQMSNVRSEDTATYFCIAHYSGGGFAYWGQGTSVTVSS.

### Variable regions of Humanized monoclonal antibody D-A10 is referred to herein as "Hz R022 (D-A10 Mab)":

VL- HzD-A10 Mab - SEQ ID NO: 14: 
DIVMTQAPLSLPVTPGEPASISCRSSKSLLYSNGYNYLYWFLQKPGQSPQLLIYYMSNLA
SGVPDRFSGSGSGTDFTLKISRVEAEDVGVYYCMQSLEYPFTFGQGTKLEIK.
VH- HzD-A10 Mab - SEQ ID NO: 15 : 
EVQLVESGGGLVQPGGSLRLSCAASGFTFSSYWMSWVRQAPGKGLVWVSDINSDGSS
TKYADSVKGRFTISRDNAKNTLYLQMNSLRAEDTAVYYCIAHYSGGGFAYWGQGTLVTV
SS.
VH- HzD-A10 Mab - SEQ ID NO: 16: 
GAAGTACAATTGGTAGAATCAGGTGGTGGTTTGGTTCAGCCAGGAGGATCACTGAGA
CTGTCCTGCGCTGCAAGCGGCTTTACCTTCTCTAGCTACTGGATGTCTTGGGTCCGG
CAAGCCCCAGGGAAGGGACTGGTGTGGGTGAGCGATATTAATAGTGACGGCTCTTC
 
TACTAAGTATGCTGATAGTGTCAAGGGCCGATTCACCATCTCACGAGACAACGCCAA
GAACACCTTGTACCTCCAGATGAACTCTTTGAGAGCTGAGGATACAGCAGTGTATTA
CTGTATCGCCCACTACTCAGGGGGAGGCTTTGCTTACTGGGGTCAAGGCACACTCG
TGACAGTCTCCTCT.
VL- HzD-A10 Mab - SEQ ID NO: 17:

GATATTGTAATGACTCAAGCTCCACTCTCCTTGCCTGTAACTCCTGGAGAGCCCGCTT
CTATTAGCTGTAGGAGTAGTAAAAGCCTGCTTTACAGTAATGGTTACAATTACCTGTA
CTGGTTTTTGCAGAAGCCTGGACAGTCACCCCAGCTCCTCATCTATTATATGTCTAAC
TTGGCCAGTGGTGTCCCAGACCGTTTTAGTGGCAGCGGCTCAGGCACCGACTTTAC
CCTTAAGATCAGCCGAGTCGAGGCTGAAGACGTAGGAGTGTACTACTGTATGCAGAG
TCTTGAGTATCCATTCACCTTCGGGCAGGGCACCAAGCTCGAAATAAAG.

### Variable regions of Monoclonal antibody m-R022 (D-D6):

VL-mR022 (D-D6) - SEQ ID NO: 35 
GATGTTGTGATGACCCAGACTCCACTCTCCCTGCCTGTCAGTCTTGGAGATCAAGCC
TCCATCTCTTGCAGATCTAGTCAGAGCCTTGTACACAGTAATGGAAACACCTATTTAC
ATTGGTACCTGCAGAAGCCAGGCCAGTCTCCAAAGGTCCTGATCTACAAAGTTTCCA
ACCGATTTTCTGGGGTCCCAGACAGGTTCAGTGGCAGTGGATCAGGGACAGATTTCA
CACTCAAGATCAGCAGAGTGGAGGCTGAGGATCTGGGAGTTTATTTCTGCTCTCAAA
GTACACATGTTCCATTCACGTTCGGTGGAGGCACCAAGCTCGAGATCAAA
VL-mR022 (D-D6) - SEQ ID NO: 36 
DVVMTQTPLSLPVSLGDQASISCRSSQSLVHSNGNTYLHWYLQKPGQSPKVLIYKVSNR
FSGVPDRFSGSGSGTDFTLKISRVEAEDLGVYFCSQSTHVPFTFGGGTKLEIK
VH-mR022 (D-D6) - SEQ ID NO: 37 
GATGTGCAGCTTCAGGAGTCGGGACCTGGCCTGGTGAAACCTTCTCAGTCTCTGTCC
CTCACCTGCACTGTCACTGGCTACTCAATCACCAGTGATTATGCCTGGAACTGGATC
CGGCAGTTTCCAGGAAACAAACTGGAGTGGATGGGCTACATAAGCTACAGTGGTCG
CACTAGCTACAACCCATCTCTCAAAAGTCGAATCTCTATCACTCGAGACACATCCAAG
AACCATTTCTTCCTGCAGTTGAATTCTGTGACTACTGAGGACACAGCCACATATTACT
GTGCCCCACTTACGACAGGGGTAGGCTATGCTATGGACTACTGGGGTCAAGGAACC
TCGGTCACCGTCTCCTCA
VH-mR022 (D-D6) - SEQ ID NO: 38

DVQLQESGPGLVKPSQSLSLTCTVTGYSITSDYAWNWIRQFPGNKLEWMGYISYSGRTS
YNPSLKSRISITRDTSKNHFFLQLNSVTTEDTATYYCAPLTTGVGYAMDYWGQGTSVTVS
S

### Variable regions of Monoclonal antibody m-R022 (D-F5):

VL-mR022 (D-F5) - SEQ ID NO: 39 
GATGTTGTGATGACTCAAACTCCACTCTCCCTGCCTGTCAGTCTTGGAGATCAAGCC
TCCATCTCTTGCAGATCTAGTCAGAGCATTGTACATAGTAATGGAAACACCTATTTAG
AATGGTACCTGCAGAAACCAGGCCAGTCTCCAAAGCTCCTGATCTACAAAGTTTCCA
ACCGATTGTCTGGGGTCCCAGACAGGTTCAGTGGCAGTGGATCAGGGACAGACTTC
ACACTCAAGATCAGCAGAGTGGAGGCTGAGGATCTGGGAGTTTATTACTGCTTTCAA
GGTTCACTTGTTCCGCTCACGTTCGGTGGAGGCACCAAGCTCGAGATCAAA
VL-mR022 (D-F5) - SEQ ID NO: 40 
DVVMTQTPLSLPVSLGDQASISCRSSQSIVHSNGNTYLEWYLQKPGQSPKLLIYKVSNRL
SGVPDRFSGSGSGTDFTLKISRVEAEDLGVYYCFQGSLVPLTFGGGTKLEIK
VH-mR022 (D-F5) - SEQ ID NO: 41 
CAGGTGCAGCTTAAGGAGTCGGGACCTGGCCTGGTGGCGCCCTCACAGAGCCTGT
CCATCACTTGCACTGTCTCTGGGTTTTCATTAACCAGCTATGGTGTACACTGGGTTC
GCCAGCCTCCAGGAAAGGGTCTGGAGTGGCTGGGAGTAATATGGGCTGGTGGAAG
CACAAATTATAATTCGGCTCTCATGTCCAGACTGCGCATCAGCAAAGACAACTCCAA
GAGCCAAGTTTTCTTAAAAATGAACAGTCTGCAAACTGATGACACAGCCATGTACTA
CTGTGCCAGAATCTATGGTAACTACGGGAGGTTTGCTTACTGGGGTCAAGGAACCT
CGGTCACCGTCTCCTCA
VH-mR022 (D-F5) - SEQ ID NO: 42

QVQLKESGPGLVAPSQSLSITCTVSGFSLTSYGVHWVRQPPGKGLEWLGVIWAGGSTN
YNSALMSRLRISKDNSKSQVFLKMNSLQTDDTAMYYCARIYGNYGRFAYWGQGTSVTV
SS

### Constant domains used in the various antibodies of the invention:

SEQ ID NO: 18 is the amino acid sequence of the constant human heavy chain : 





.
SEQ ID NO: 19 is the nucleotide sequence of the constant human heavy chain: 
GCCTCCACCAAGGGCCCATCGGTCTTCCCCCTGGCACCCTCCTCCAAGAGCACC
TCTGGGGGCACAGCGGCCCTGGGCTGCCTGGTCAAGGACTACTTCCCCGAACCG
GTGACGGTGTCGTGGAACTCAGGCGCCCTGACCAGCGGCGTGCACACCTTCCCGG
 
CTGTCCTACAGTCCTCAGGACTCTACTCCCTCAGCAGCGTCGTGACCGTGCCCTCC
AGCAGCTTGGGCACCCAGACCTACATCTGCAACGTGAATCACAAGCCCAGCAAC
ACCAAGGTGGACAAGAAAGTTGAGCCCAAATCTTGTGACAAAACTCACACATGCCCA
CCGTGCCCAGCACCTGAACTCCTGGGGGGACCGTCAGTCTTCCTCTTCCCCCCA
AAACCCAAGGACACCCTCATGATCTCCCGGACCCCTGAGGTCACATGCGTGGTGGT
GGACGTGAGCCACGAAGACCCTGAGGTCAAGTTCAACTGGTACGTGGACGGCGTG
GAGGTGCATAATGCCAAGACAAAGCCGCGGGAGGAGCAGTACAACAGCACGTACC
GTGTGGTCAGCGTCCTCACCGTCCTGCACCAGGACTGGCTGAATGGCAAGGAGTAC
AAGTGCAAGGTCTCCAACAAAGCCCTCCCAGCCCCCATCGAGAAAACCATCTCCAA
AGCCAAAGGGCAGCCCCGAGAACCACAGGTGTACACCCTGCCCCCATCCCGGGAT
GAGCTGACCAAGAACCAGGTCAGCCTGACCTGCCTGGTCAAAGGCTTCTATCCCAG
CGACATCGCCGTGGAGTGGGAGAGCAATGGGCAGCCGGAGAACAACTACAAGACC
ACGCCTCCCGTGCTGGACTCCGACGGCTCCTTCTTCCTCTACAGCAAGCTCACCGT
GGACAAGAGCAGGTGGCAGCAGGGGAACGTCTTCTCATGCTCCGTGATGCATGAG
GCTCTGCACAACCACTACACGCAGAAGAGCCTCTCCCTGTCTCCGGGTAAATGA.
SEQ ID NO: 20 is the amino acid sequence of the constant human light chain used in the various antibodies of the invention: 

.
SEQ ID NO: 21 is the nucleotide sequence of the constant human light chain used :

CGAACTGTGGCTGCACCATCTGTCTTCATCTTCCCGCCATCTGATGAGCAGTTGAAA
TCTGGAACTGCCTCTGTTGTGTGCCTGCTGAATAACTTCTATCCCAGAGAGGCCAAA
GTACAGTGGAAGGTGGATAACGCCCTCCAATCGGGTAACTCCCAGGAGAGTGTCAC
AGAGCAGGACAGCAAGGACAGCACCTACAGCCTCAGCAGCACCCTGACGCTGAGC
AAAGCAGACTACGAGAAACACAAAGTCTACGCCTGCGAAGTCACCCATCAGGGCCT
GAGCTCGCCCGTCACAAAGAGCTTCAACAGGGGAGAGTGTTAG.

### The invention will now be described more in detail using non-limiting examples referring to the figures.

### BRIEF DESCRIPTION OF THE TABLES AND DRAWINGS

### Target expression and HzR022 (D-A10) epitope detection

**Table 1** illustrates HzR022 (D-A10) cellular staining analyzed by flow cytometry. The mean + - SD on percentage of labelled cells (%) and Mean of intensity (MFI) are shown on different cell line cancers such as colorectal (CRC), Head & Neck (HAN), Glioblastoma (GBM), Urinary (U), Prostate (PC), Breast (B), Lung (L), Pancreatic (PRC) or Renal (R).

**Table 2** illustrates HzR022 (D-A10) cellular staining analyzed by Immunohistochemistry on Colorectal PDX xenograft models

**Table 3** illustrates HzR022 (D-A10) cellular staining analyzed by Immunohistochemistry on Pancreas PDX xenograft models

### In vivo proof of concepts

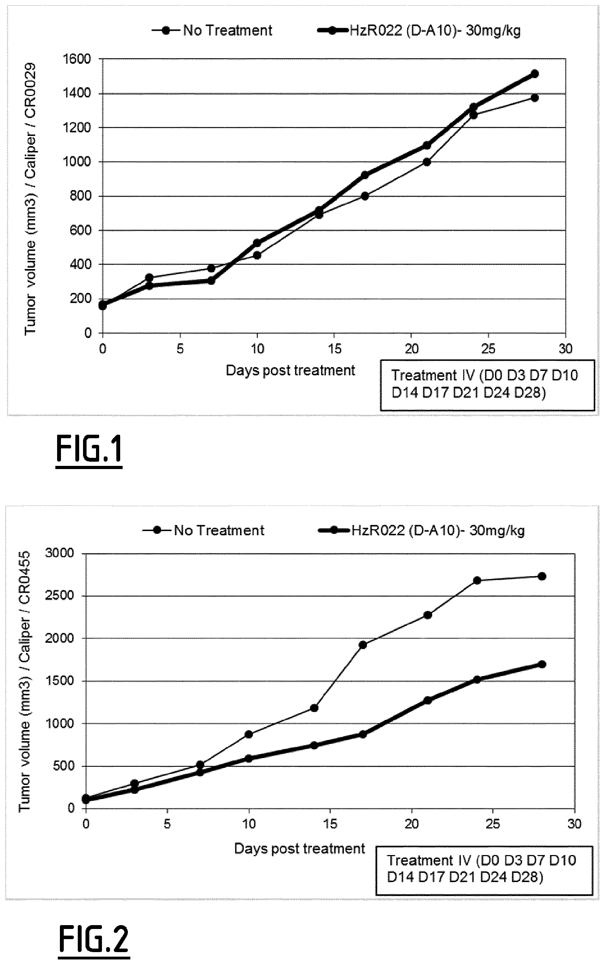

**Figure 1** illustrates *in vivo* inhibition of tumour growth of PDX xenograft CR0029 model CRC K-ras wild type with MAb anti-eCK8 / HzR022 (D-A10) - representative experiment.
**Figure 2** illustrates *in vivo* inhibition of tumour growth of PDX xenograft CR0455 model CRC K-ras mutated with MAb anti-eCK8 / HzR022 (D-A10) - representative experiment.
   **Table 4** shows a review of *in vivo* inhibition of tumour growth of PDX xenograft models CRC K-ras wild type with MAb anti-eCK8 /HzR022 (D-A10) - Review on 5 PDX models.
   **Table 5** shows a review of *in vivo* inhibition of tumour growth of PDX xenograft models CRC K-ras mutated with MAb anti-eCK8 /HzR022 (D-A10) - Review on 6 PDX models.

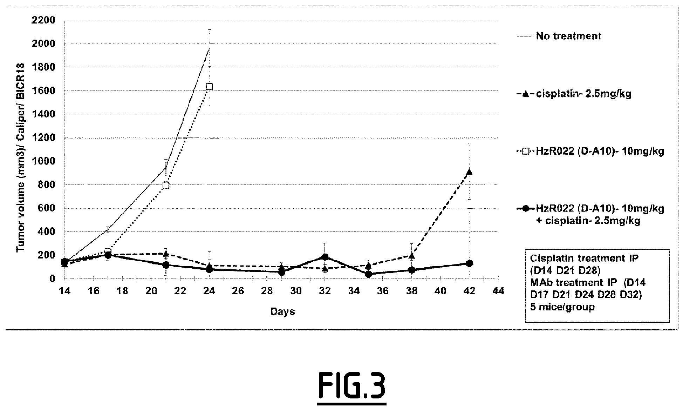

**Figure 3** illustrates *In vivo* inhibition of tumor growth of Head & Neck cancer CDX model such as Larynx cancer with MAb anti-eCK8 / HzR022 (D-A10) in combination with cisplatin from the cell line BICR18.

### MAb ability for internalisation related to Antibody drug conjugated

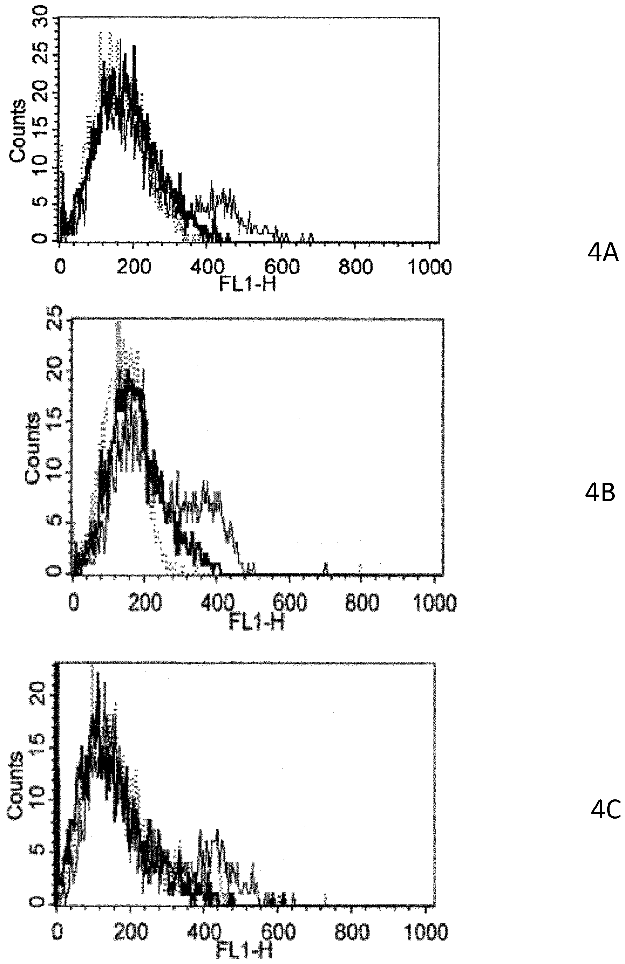

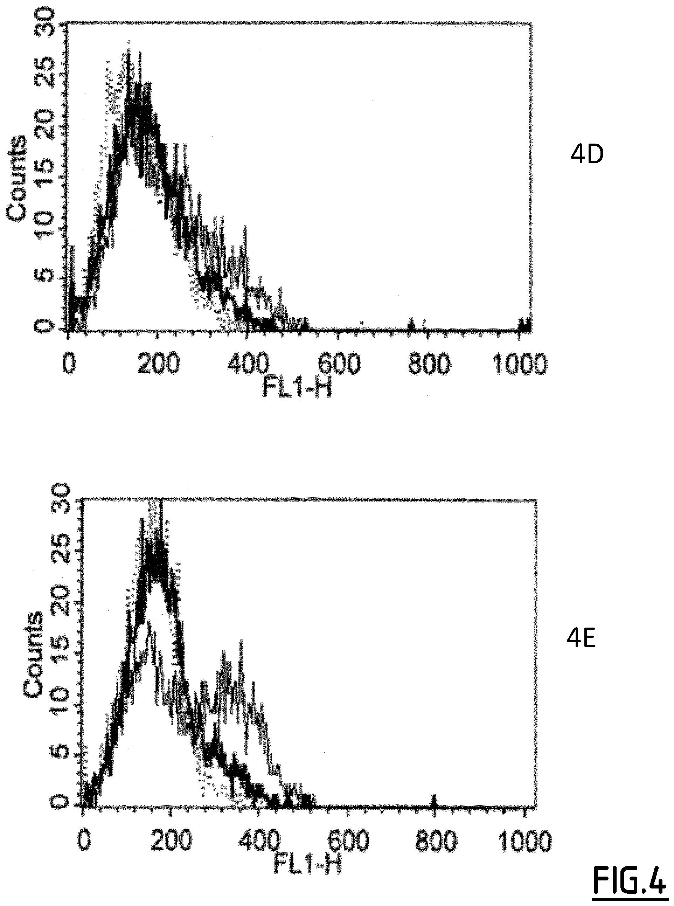

**Figure 4** illustrates D-A10 MAb internalization for solid tumors as Colorectal cancer from the cell line HCT116 (A), Glioblastoma cancer from the cell line U87MG (B), Head & Neck cancer from the cell line BiCR56 (C), Pancreas cancer from the cell line BxPC3 (D), Prostate cancer from the cell line DU145 (E). Fine line: 4°C - Bold line: 37°C - Dotted line: untreated.

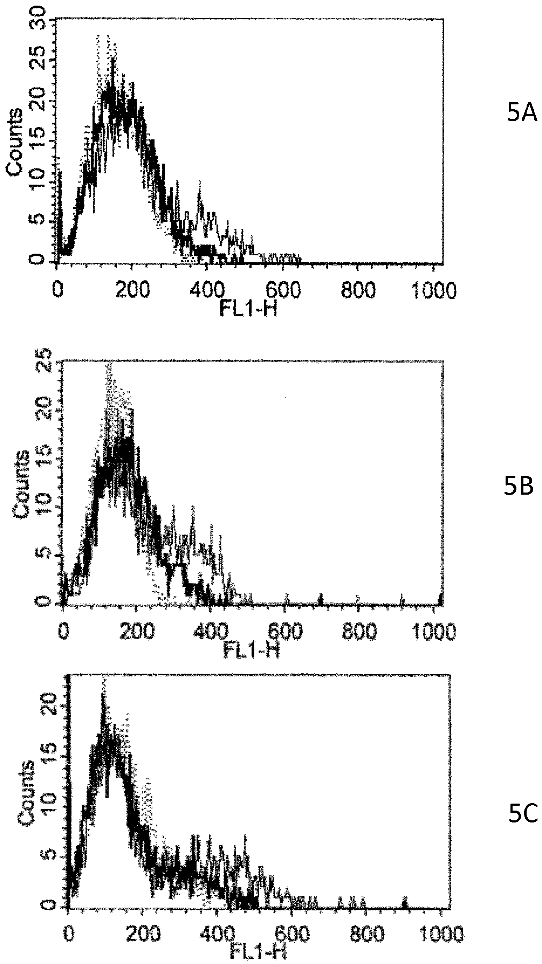

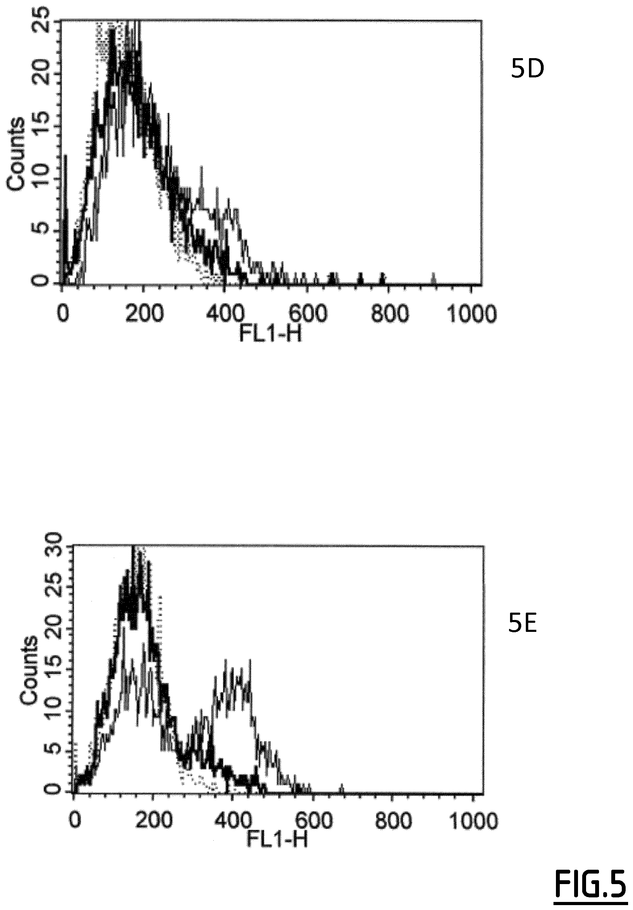

**Figure 5** illustrates D-D6 MAb internalization for solid tumors for solid tumors as Colorectal cancer from the cell line HCT116 (A), Glioblastoma cancer from the cell line U87MG (B), Head & Neck cancer from the cell line BiCR56 (C), Pancreas cancer from the cell line BxPC3 (D), Prostate cancer from the cell line DU145 (E). Fine line: 4°C - Bold line: 37°C - Dotted line: untreated.

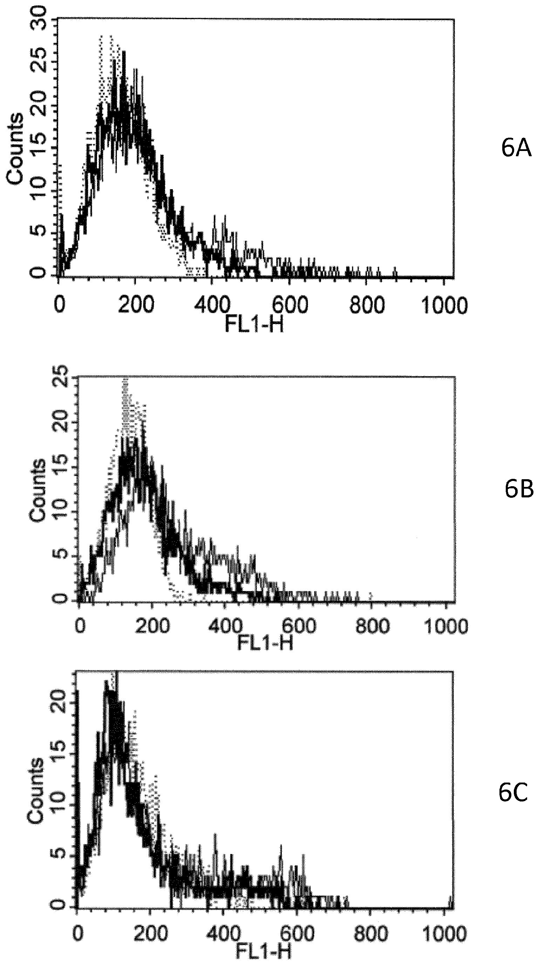

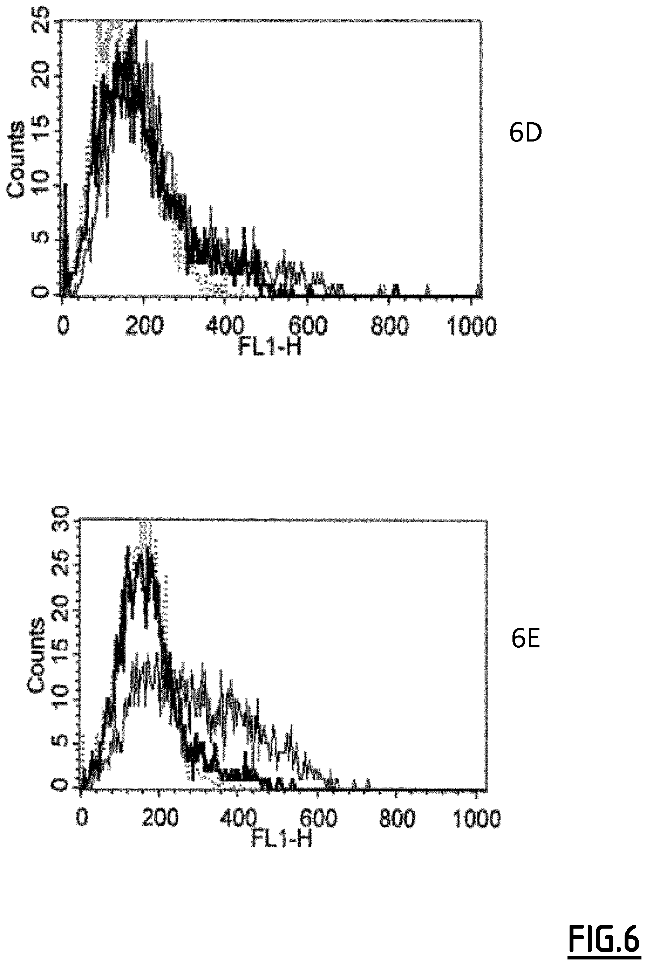

**Figure 6** illustrates D-F5 MAb internalization for solid tumors as Colorectal cancer from the cell line HCT116 (A), Glioblastoma cancer from the cell line U87MG (B), Head & Neck cancer from the cell line BiCR56 (C), Pancreas cancer from the cell line BxPC3 (D), Prostate cancer from the cell line DU145 (E)..Fine line: 4°C - Bold line: 37°C - Dotted line: untreated.

### EXAMPLES

The following examples are offered to illustrate, but not to limit the claimed invention.

### Example 1: Preparation of murine anti-CK8 antibody

The murine monoclonal antibodies specific for CK8 were produced using standard hybridoma techniques (Zola et al., Aust J. Exp Biol Med Sci. 1981; 59:303-6). Two different CK8 related peptides were synthesized and used for mice immunization as described in WO 2016/020553. After hybridoma cloning, three murine Mabs were obtained called mD-A10, mD-F5 and mD-D6. Each clone was injected into the peritoneum of nude mice. Protein A chromatography from murine ascitic fluid. The murine ascitic fluid is adjusted at pH 8.3 with the equilibration buffer 0.1 M Tris and 1.5 M Sulfate Ammonium and then loaded onto the rProtein A Sepharose Fast Flow column (GE Healthcare, Saint Cyr au Mont d'or, France). The non-binding proteins are flowed through and removed by several washings with equilibration buffer. The MAb anti-CK8 is eluted off the Protein A column using the elution buffer 0.1 M Citrate Sodium at pH 3.5. After concentration, the PBS solution containing IgG was filtered and the Mab concentration was determined at 280 nm

### EXAMPLE 2: HzR022 (D-A10) MAb production and protein A purification

Mammalian cells are the preferred hosts for production of therapeutic glycoproteins, due to their capability to glycosylate proteins in the most compatible form for human applications *(*Jenkins et al., Nat Biotech. 1996; 14:975-81*).* Mammalian host cells that could be used include, human Hela, 283, H9 and Jurkat cells, mouse NIH3T3 and C127 cells, Cos 1, Cos 7 and CV1 African green monkey cells, quail QC1-3 cells, mouse L cells and Chinese hamster ovary cells. Bacteria very rarely glycosylates proteins, and like other type of common hosts, such as yeasts, filamentous fungi, insect and plant cells yield glycosylation patterns associated with rapid clearance from the blood stream.

The Chinese hamster ovary (CHO) cells allow consistent generation of genetically stable, highly productive clonal cell lines. They can be cultured to high densities in simple bioreactors using serum-free media, and permit the development of safe and reproducible bioprocesses. Other commonly used animal cells include baby hamster kidney (BHK) cells, NSO- and SP2/0-mouse myeloma cells. Production from transgenic animals has also been tested *(*Jenkins et al., Nat Biotech. 1996; 14:975-81*).*

A typical mammalian expression vector contains the promoter element (early and late promoters from SV40, the long terminal repeats (LTRs) from Retroviruses e.g. RSV, HTLV1, HIV1 and the early promoter of the cytomegalovirus (mCMV, hCMV), which mediates the initiation of transcription of mRNA, the protein coding sequence, and signals required for the termination of transcription and polyadenylation of the transcript (BGH polyA, Herpes thimidine kinase gene of Herpes simplex virus polyA (TKpa), Late SV40 polyA and 3' UTR_Beta_Globin_polyA). Additional elements include enhancers (Eµ, hlE1), Kozak sequences, signal peptide and intervening sequences flanked by donor and acceptor sites for RNA splicing. Suitable expression vectors for use in practise in practising the present invention include, for examples, vectors such as pcDNA3.1, pcDNA3.3, pOptiVEC, pRSV, pEµMCMV, pMCMVHE-UTR-BG, pHCMVHE-UTR-BG, pMCMV-UTR-BG, pHCMV-UTR-BG, pMCMVHE-SV40, pHCMVHE-SV40, pMCMV-SV40, pHCMV-SV40, pMCMVHE-TK, pHCMVHE-TK, pMCMV-TK, pHCMV-TK, pMCMVHE-BGH, pHCMVHE-BGH, pMCMV-BGH, pHCMV-UTR-BGH).

The empty CHO Easy C cells (purchased by the CCT collection) were co-transfected with MAb expression vector for light and heavy chains following transient or stable transfection procedure established in our laboratory. Secretion of H and L chains were enabled by the respective human IgH leader sequence. The coding regions for light and heavy chains of MAb anti-CK8 are introduced into the MAb expression vector in the multiple cloning site. The transformants are analysed for correct orientation and reading frame, the expression vector may be transfected into CHO cell line.

Protein A chromatography from harvested CHO cell culture fluid. The harvested cell culture fluid produced from CHO cells is loaded onto the Hi Trap rProtein A column (GE Healthcare, Saint Cyr au Mont d'Or, France) that is equilibrated with Phosphate buffered saline, pH 7.2. The non-binding proteins are flowed through and removed by several washings with PBS buffer followed. The MAb anti-CK8 is eluted off the Protein A column using a step of elution of 0.1 M Citric acid at pH 3.0. Column eluent is monitored by A280. The anti-CK8 MAb peak is pooled.

### EXAMPLE 3: Cell culture

Various tumour-derived cell lines are among the target cells that may be stained with MAb anti-CK8, in such assay procedures.

Cell lines. The established human neuroglioma cells H4, HS683, U373 or A172 (available from ATCC); the established human colorectal cells HT29; the established human pancreatic cells PANC1 or MIA-PA-CA2; the established human kidney adenocarcinoma cells A704 or ACHN and the established human lung adenocarcinoma cells A549 were grown in Dulbecco's Modified Eagle's Medium (Sigma, St Quentin Fallavier, France) supplemented with 10% heat-inactivated foetal bovine serum (FBS) (Sigma, St Quentin Fallavier, France), 4 nM L-glutamine (Sigma, St Quentin Fallavier, France) and 100 U/mL, 100 µg/mL penicillin - streptomycin (Sigma, St Quentin Fallavier, France). The established human glioblastoma astrocytoma cells U87MG or T98G, the human head and neck cancer cells FaDu or Detroit562, the human urinary cancer cells UM-UC-3, J82, HT1197 or HT1376 and the human prostate cancer cells DU145 were grown in Eagle's Minimum Essential Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin - streptomycin (Sigma, St Quentin Fallavier, France). The established human breast adenocarcinoma cells MDAMB231, MCF-7 or HBL100 and the human colorectal cancer cells HCT116 were grown in Dulbecco's Modified Eagle's Medium Glutamax Low Glucose (Life Technologies, St Aubin, France) supplemented with 10% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin - streptomycin (Sigma, St Quentin Fallavier, France).The established human colorectal cells HCT15 or SW480 and the human head and neck cancer cells TR146 were grown in Dulbecco's Modified Eagle's Medium Glutamax High Glucose (Life Technologies, St Aubin, France) supplemented with 10% heat-inactivated fetal bovine serum (FBS) (Sigma, St Quentin Fallavier, France) and 100 U/mL, 100 µg/mL penicillin - streptomycin (Sigma, St Quentin Fallavier, France). The established human head and neck cancer cells BICR16, BICR18 or BICR56 were grown in Dulbecco's Modified Eagle's Medium (Sigma, St Quentin Fallavier, France) supplemented with 10% heat-inactivated foetal bovine serum (FBS) (Sigma, St Quentin Fallavier, France), 4 nM L-glutamine (Sigma, St Quentin Fallavier, France), 100 U/mL, 100 µg/mL penicillin - streptomycin (Sigma, St Quentin Fallavier, France) and 0,4 µg/mL hydrocortisone (Sigma, St Quentin Fallavier, France). The established human head and neck cancer cells SCC9, SCC4 or SCC15 were grown in Dulbecco's Modified Eagle's Medium/ F12 (Life Technologies, St Aubin, France) supplemented with 10% heat-inactivated foetal bovine serum (FBS) (Sigma, St Quentin Fallavier, France), 4 nM L-glutamine (Sigma, St Quentin Fallavier, France), 100 U/mL, 100 µg/mL penicillin - streptomycin (Sigma, St Quentin Fallavier, France) and 0,4 µg/mL hydrocortisone (Sigma, St Quentin Fallavier, France). The established human urinary bladder carcinoma cells 5637, the human prostate cancer cells LNCap clone FGC, the established human lung adenocarcinoma cells NCIH1703 or NCIH292, the human pancreatic cancer cells PSN1 or BxPC3 and the human kidney adenocarcinoma cells Caki1 were grown in RPMI-1640 Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin -streptomycin (Sigma, St Quentin Fallavier, France). The established human glioma cells 42MGBA (available from DSMZ) were grown in 80% mixture of RPMI-1640 Medium and Eagle's Minimum Essential Medium at 1:1 (Sigma, St Quentin Fallavier, France) supplemented with 20% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin -streptomycin (Sigma, St Quentin Fallavier, France). The established human glioma cells 8MGBA were grown in Eagle's Minimum Essential Medium (Sigma, St Quentin Fallavier, France) supplemented with 20% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin - streptomycin (Sigma, St Quentin Fallavier, France). The established human urinary cancer cells TCCSUP and the human pancreatic cancer cells HuPT3 were grown in Eagle's Minimum Essential Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France), 100 U/mL, 100 µg/mL penicillin - streptomycin (Sigma, St Quentin Fallavier, France), 1% sodium pyruvate (Sigma, St Quentin Fallavier, France) and 1% non-essential amino acid (Sigma, St Quentin Fallavier, France). The human pancreatic cancer cells AsPC1 were grown in RPMI-1640 Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France), 100 U/mL, 100 µg/mL penicillin -streptomycin (Sigma, St Quentin Fallavier, France) and 1% sodium pyruvate (Sigma, St Quentin Fallavier, France). The human pancreatic cancer cells CFPAC1 were grown in Iscove's Modified Dulbecco's Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin -streptomycin (Sigma, St Quentin Fallavier, France).

### EXAMPLE 4: Antibody binding assays by Flow cytometry

This example describes methods to investigate on CK8 cellular expression at the cell surface analysed by flow cytometry.

Flow cytometry experiments for CK8 cellular expression. Briefly, 2.10⁵ cells per 96 wells are incubated at 4°C with a dilution of unconjugated humanised anti CK8 HZMR022/D-A10 MAb at 5 µg/mL then diluted at 1/2. The negative control MAb used was human IgG1 kappa (Sigma, St Quentin Fallavier, France). Unbound antibodies were washed away with PBS (Life technologies, St Aubin, France) supplemented by 1% Bovine Serum Albumin (Sigma, St Quentin Fallavier, France). Subsequently, cells are centrifuged (5 min at 400 g) and bound antibody is detected with Phycoerythrin (PE) conjugated Goat anti human IgG (Sigma, St Quentin Fallavier, France) at 4°C for 30 min. Detection reagent is washed away and cells are centrifuged (5 min at 400 g) and suspended in 300 µL PBS. Bound detection antibody is quantified on a FACSCAN (BD Biosciences, Rungis, France), (FL2 channel, 2000 events per acquisition). During the experiment, the respective isotype controls are included to exclude any unspecific binding events.

Results of experiments are shown in TABLE 1 (at 5 µg/mL). Various cancer cell lines express CK8 epitope identified with HZMR022/D-A10. Expression patterns varied from cell line to cell lines. In the present study CK8 was expressed on all cell lines tested, among a cell subset.

### EXAMPLE 5: Antibody binding assays by Immunohistochemistry

This example describes methods to investigate on CK8 cellular expression at the cell surface by Immunohistochemistry. The antigen retrieval (AR) was performed following incubation at 95°C during 30 min in Sodium Citrate (pH6.0). The MAb anti CK8 was incubated 1 hour at room temperature (RT). The secondary antibody as a goat anti human was incubated at RT during 1 hour then with a rabbit polyclonal anti FITC at RT during 30 min. Then the MAb IHC staining was revealed by using an ultravision LP detection system (primary antibody enhancer 10 min, HRP Polymer, 15 min) at RT following by an incubation with DAB at RT during 3 min.

Eleven CRC PDX models were evaluated as CR0004, CR0012, CR0029, CR0126, CR0196, CR0205, CR0455, CR1530, CR3056, CR3150 and CR6254.

Results of experiments are shown in TABLE 2. All of CRC PDX models expressed the HzR022 (D-A10) epitope detected by IHC. Different IHC scores were observed as 2+ for strong or 1 + for moderate intensity IHC staining.

Four PC PDX models were evaluated as PAN-001, PAN-003, PAN-004 and PAN-035.

Results of experiments are shown in TABLE 3. All of PC PDX models expressed the HzR022 (D-A10) epitope detected by IHC. The IHC score was determined according the membranous and cytoplasmic intensity. The IHC score of 4 was observed for all of them revealing a strong to very strong IHC staining.

### EXAMPLE 6: In vivo investigation on PDX models for CRC

Each mouse was inoculated subcutaneously at the right flank with one primary human tumour xenograft model (CR00004, CR0012, CR0029, CR0126, CR0196, CR0205, CR0455, CR1530, CR3056, CR3150 or CR6254) tumour fragment (2-3 mm in diameter) for tumour development. When average or individual tumour size reaches 100-250 mm3, mice was randomly (rolling enrollment will be involved if necessary) allocated into 4 groups. Each group contained 1 mouse. The day of grouping and dosing initiation was denoted as day 0. The dosing volume was adjusted for body weight (Dosing volume = 5 µL/g). After tumour inoculation, the animals were checked daily for morbidity and mortality. At the time of routine monitoring, the animals were checked for any effects of tumour growth and treatments on normal behavior such as mobility, food and water consumption, body weight gain/loss, eye/hair matting and any other abnormal effect. Death and observed clinical signs were recorded on the basis of the numbers of animals within each subset. Two weeks of dosing-free observation were applied after final treatment. The animals in vehicle group were sacrificed before study termination because of tumour volume (TV) over 3000mm³. Tumour size was measured by caliper twice weekly in two dimensions. The tumour volume was expressed in mm3 using the formula: TV = 0.5 a × b2 where a and b are the long and short diameters of the tumour, respectively. Body weight was measured twice weekly. When individual mouse has a body weight loss ≥15%, the mouse was given dosing holiday(s) until its body weight recovers to body weight loss. Under following conditions, the in-life experiment of individual animal or whole groups was terminated, by human euthanasia, prior to death, or before reaching a comatose state.

**Study design for MAb impact regarding CRC subtypes**

| **Group** | **N** | **Treatment** | **Dose level (mg/kg)** | **Dose Route** | **Dosing Frequency** |
|---|---|---|---|---|---|
| 1 | 1 | No treatment | NA | NA | NA |
| 2 | 1 | HzR022 (D-A10) MAb anti CK8 | 30 | *i.v.* | BIW×4 |

| | | | | | |
|---|---|---|---|---|---|
| *Study design of MAb impact- N: animal number per group* | | | | | |

### K-Ras wild type in vivo proof of concept from CRC patient derivate (PDX models)

5 HuPrime^{®} CRC K-ras wt xenograft models (selected in CRO cell bank after CK8 IHC positive detection) were selected as CR0004, CR0029, CR0196, CR0205 or CR3056.

Excepted the CRC PDX K-Ras wt mode CR0205, all of K-Ras wt CRC did not respond to HzR022 (D-A10) MAb. No control of tumour progression was observed at 30 mg/kg among 4/5 CRC PDX K-ras wt models as illustrated in Table 4.

### K-Ras mutated in vivo proof of concept from CRC patient derivate (PDX models)

6 HuPrime^{®} CRC K-Ras mutated xenograft models (selected in CRO cell bank after CK8 IHC positive detection) were selected as CR0012, CR0126, CR0455, CR1530, CR3150 or CR6254. HzR022 (D-A10) mediated tumour progression was observed at 30 mg/kg among 6/6 CRC PDX K-Ras mutated models as illustrated in Table 5.

### Example 7: In vivo investigation on HAN CDX model

Human HAN larynx cell line BICR18 was subcutaneously injected in SCID mice, with a concentration of 1.10⁶ cells per injection (200 µL). Mice were randomized when the tumours reached a mean volume of about 100 mm³ for the 9 groups (total 45 mice). All the mice were observed in order to detect any toxic effects of the product. The endpoint was defined by animal ethics as a tumour diameter of >18mm, significant weight loss or alteration of animal well-being. In order to assess the effectiveness of the compounds on tumourigenesis, tumour volume was measured two times a week. The size of the primary tumours were measured using calipers and the tumour volume (TV) was extrapolated to a sphere using the formula TV= 4/3 π x r³, by calculating the mean radius from the two measurements. The median and standard deviation were also calculated for each group. Median is preferred to mean in order to exclude the extreme values. MAb treatment was administered by intraperitoneal injection twice a week during three weeks at 10 or 1 mg/kg doses. The cisplatin (Myland) treatment was administered by intraperitoneal injection once per week during three weeks at 1, 2.5 or 5 mg/kg doses. The product was prepared in accordance with the sponsor's guidelines, i.e. diluted in PBS. Mice were sacrificed when the tumours reached a maximum volume of 1600 mm³. The endpoints were defined by clinical trial ethics as a tumour diameter of >18mm or weight loss of >10% of body weight, or when the tumours are dangerous for mice (necrosis). Statistical analysis was performed with GraphPad Prism software. GraphPad Prism combined scientific graphing, comprehensive curve fitting, understandable statistics, and data organization. The t-test (two-tailed test) was performed on the tumour volume values (mm³) measured on the day of sacrifice.

**Study design for MAb impact on HAN CDX**

| **Group** | **N** | **Treatment** | **Dose level (mg/kg)** | **Dose Route** | **Dosing Frequency** |
|---|---|---|---|---|---|
| 1 | 5 | No treatment | - | - | |
| 2 | 5 | HzR022 (D-A10) | 10 | *i.p.* | BIW×3 |
| 3 | 5 | Cisplatin | 2.5 | *i.p.* | QW×3 |
| 4 | 5 | HzR022 (D-A10) + Cisplatin | 10 | *i.p.* | BIW×3 |
| | | | 2.5 | | QW×3 |

| | | | | | |
|---|---|---|---|---|---|
| *Study design of MAb impact* *N: animal number per group* | | | | | |

Results shown in Figure 3 that the combination treatment resulted in synergic cytotoxicity of tumour growth of HAN with MAb anti-CK8 / HzR022 (D-A10) in combination with cisplatin. Although HzR022 (D-A10) alone did not trigger tumour growth control, the combination of HzMR022/D-A10 and cisplatin reverse the escape of tumour cisplatin sensitivity.

### EXAMPLE 8: Antibody internalisation assays by flow cytometry

This example describes methods to investigate on HzR022 (D-A10) internalisation following CK8 detection at the cell surface analysed by flow cytometry.

Flow cytometry experiments for MAb internalisation. Briefly, 2.10⁵ cells per 96 wells are incubated at 4°C versus 37°C during 24 hours with a dilution of unconjugated murine anti CK8 MAb at 50 µg/mL then diluted at 1/2. The MAb anti CK8 tested were D-A10 (IgG2b), D-F5 (IgG2a) or D-D6 (IgG1), from iDD biotech MAb panel. The isotype matched MAbs used were B-Z1 (IgG1), B-Z2 (IgG2a) or B-E4 (IgG2b) (Diaclone, Besançon, France). Unbound antibodies were washed away with PBS (Life technologies, St Aubin, France) supplemented by 1% Bovine Serum Albumin (Sigma, St Quentin Fallavier, France). Subsequently, cells are centrifuged (5 min at 400 g) and bound antibody is detected with Fluorescein Isothiocyanate (FITC) conjugated goat (Fab')₂ polyclonal anti mouse Ig (MP Biomedical, Illkirch, France) at 4°C for 30 min. Detection reagent is washed away and cells are centrifuged (5 min at 400 g) and suspended in 300 µL PBS. Bound detection antibody is quantified on a FACSCAN (BD Biosciences, Rungis, France), (FL1 channel, 3 000 events per acquisition). During the experiment, the respective isotype controls are included to exclude any unspecific binding events
Results of experiments are shown in Figure 4 for D-A10 MAb (at 0.78 or 0.39 µg/mL), in Figure 5 for D-D6 (at 0.78 or 0.39 µg/ml), in Figure 6 for D-F5 (at 0.78 or 0.39 µg/ml). One example was shown for CRC from HCT116 cell line, GBM from U87MG cell line, HAN from BIRC56 cell line, PC from BxPC3 or PRC from DU145.

Whatever the MAb D-F5 exhibited a lower internalization potential, a highest internalization potential was observed for D-A10 or D-D6 MAb anti-CK8.

**Table 1 : eCK8 expression on solid tumours**

| **Cancer** | **Cell line** | **% labelled cells** | **MFI** | **Expt (n=)** |
|---|---|---|---|---|
| Colon | HT29 | 49 ±9 | 203±22 | 2 |
| | HCT116 | 64 ±0 | 283±16 | 2 |
| | SW480 | 38 ±3 | 179±1 | 2 |
| Head & Neck | FaDu | 56 ±8 | 258±20 | 4 |
| | Detroit562 | 36 ±0 | 203±15 | 2 |
| | BICR16 | 52 ±2 | 206+10 | 2 |
| | BICR18 | 79±9 | 257±23 | 2 |
| | BICR56 | 39±34 | 123±4 | 3 |
| | SCC9 | 30±23 | 193±23 | 3 |
| | SCC4 | 59±16 | 222±88 | 4 |
| | SCC 15 | 41±21 | 167±13 | 2 |
| | TR146 | 55±18 | 140±4 | 2 |
| GBM | H4 | 58±33 | 199±40 | 4 |
| | A172 | 35±23 | 186±14 | 4 |
| | U373 | 53±1 | 161±7 | 4 |
| | 8-MG-BA | 72±11 | 261±38 | 4 |
| | 42-MG-BA | 54±7 | 247±44 | 4 |
| | T98G | 48±25 | 211±20 | 4 |
| | HS683 | 54±9 | 225±24 | 4 |
| | U87-MG | 38±23 | 178±35 | 4 |
| Urinary | 5637 | 59±20 | 284±61 | 4 |
| | UM-UC-3 | 26±23 | 156±14 | 2 |
| | J82 | 68±9 | 319±4 | 2 |
| | TCCSUP | 62±10 | 255±48 | 2 |
| | HT1197 | 23±7 | 240±4 | 2 |
| | HT1376 | 53±3 | 497±11 | 2 |
| Prostate | DU145 | 19±11 | 185±42 | 4 |
| | LNCaP clone FGC | 76±9 | 412±21 | 2 |
| Breast | MCF-7 | 56±33 | 250±61 | 2 |
| | MDAMB231 | 49±14 | 179±15 | 3 |
| | HBL100 | 67±8 | 219±6 | 2 |
| Lung | A549 | 36±12 | 193±38 | 5 |
| | NCIH1703 | 33±10 | 188±12 | 4 |
| | NCIH292 | 53±20 | 272±38 | 4 |
| Pancreatic | HuP-T3 | 62±17 | 260±20 | 2 |
| | MIA-Pa-Ca-2 | 58±14 | 264±14 | 4 |
| | PANC-1 | 37±7 | 198±23 | 4 |
| | PSN-1 | 15±6 | 193±16 | 4 |
| | AsPC-1 | 26±14 | 181±17 | 4 |
| | BxPC-3 | 41±17 | 220±17 | 4 |
| | CFPAC-1 | 62±4 | 279±25 | 4 |
| Renal | ACHN | 46±20 | 195±20 | 4 |
| | A704 | 37±26 | 184±30 | 4 |
| | Caki1 | 49±12 | 267±36 | 4 |

**Table 2: HzR022 (DA-10) cellular staining analyzed by Immunohistochemistry on Colorectal PDX xenograft models**

| **CRC PDX model** | **HzR022 (D-A10) IHC score** |
|---|---|
| CR0004 | 2+ |
| CR0012 | 2+ |
| CR0029 | 2+ |
| CR0126 | 2+ |
| CR0196 | 2+ |
| CR0205 | 1+ |
| CR0455 | 1+ |
| CR1530 | 2+ |
| CR3056 | 1+ |
| CR3150 | 1+ |
| CR6254 | 2+ |

**Table 3: HzR022 (D-A10) cellular staining analyzed by Immunohistochemistry on Pancreas PDX xenograft models**

| **PC PDX model** | **Membraneous Intensity** | **Cytoplasmic Intensity** | **HZMR022/D-A10 IHC score** |
|---|---|---|---|
| PAN-001 | 4 | 4 | 16 |
| PAN-003 | 4 | 4 | 16 |
| PAN-004 | 4 | 4 | 16 |
| PAN-035 | 4 | 4 | 16 |

**Table 4: In vivo inhibition of tumour growth of PDX xenograft model CRC K-Ras wild type with MAb anti-eCK8 / HzR022 (D-A10) - Review on 5 PDX models**

| **CRC PDX model** | **K-RAS genomic profile** | **HzR022 (D-A10) activity** |
|---|---|---|
| CR0004 | wild type | NR |
| CR0029 | wild type | NR |
| CR0196 | wild type | NR |
| CR0205 | wild type | R |
| CR3056 | wild type | NR |

| | | |
|---|---|---|
| NR: Non responder to HzR022 (D-A10) MAb R: Responder to HzR022 (D-A10) MAb | | |

**Table 5: In vivo inhibition of tumour growth of PDX xenograft model CRC K-Ras mutated with MAb anti-eCK8 / HzR022 (D-A10) - Review on 6 PDX model**

| **CRC PDX model** | **K-RAS genomic profil** | **HzR022 (D-A10) activity** |
|---|---|---|
| CR0012 | G13D | R |
| CR0126 | G12V | R |
| CR0455 | G12D | R |
| CR1530 | Q61H | R |
| CR3150 | G12V | R |
| CR6254 | A146T | R |

| | | |
|---|---|---|
| NR: Non responder to HzR022 (D-A10) MAb R: Responder to HzR022 (D-A10) MAb | | |

## Claims

1. An anti-CK8 monoclonal antibody selected from:
- antibody comprising a heavy chain comprising the following three CDRs, respectively CDR-H1, CDR-H2 and CDR-H3, wherein CDR-H1 comprises the sequence SEQ ID NO: 8; CDR-H2 comprises the sequence SEQ ID NO: 9; and CDR-H3 comprises the sequence SEQ ID NO: 10.; and a light chain comprising the following three CDRs, respectively CDR-L1, CDR-L2 and CDR-L3, wherein CDR-L1 comprises the sequence SEQ ID NO: 5; CDR-L2 comprises the sequence SEQ ID NO: 6; and CDR-L3 comprises the sequence SEQ ID NO: 7;
- antibody comprising a heavy chain comprising the following three CDRs, respectively CDR-H1', CDR-H2' and CDR-H3', wherein CDR-H1' comprises the sequence SEQ ID NO: 11; CDR-H2' comprises the sequence SEQ ID NO: 12; and CDR-H3' comprises the sequence SEQ ID NO: 10; and a light chain comprising the following three CDRs, respectively CDR-L1', CDR-L2' and CDR-L3', wherein CDR-L1' comprises the sequence SEQ ID NO: 13; CDR-L2' comprises the sequence SEQ ID NO: 6; and CDR-L3' comprises the sequence SEQ ID NO: 7;
for its use in treating a tumour expressing CK8 and having at least one of the following mutations in K-Ras: G13D, G12V, G12D, G61H, G12V or A146T.

2. An anti-CK8 monoclonal antibody selected from :
- antibody comprising a heavy chain comprising the following three CDRs, respectively CDR-H1, CDR-H2 and CDR-H3, wherein CDR-H1 comprises the sequence SEQ ID NO: 8; CDR-H2 comprises the sequence SEQ ID NO: 9; and CDR-H3 comprises the sequence SEQ ID NO: 10.; and a light chain comprising the following three CDRs, respectively CDR-L1, CDR-L2 and CDR-L3, wherein CDR-L1 comprises the sequence SEQ ID NO: 5; CDR-L2 comprises the sequence SEQ ID NO: 6; and CDR-L3 comprises the sequence SEQ ID NO: 7;
- antibody comprising a heavy chain comprising the following three CDRs, respectively CDR-H1', CDR-H2' and CDR-H3', wherein CDR-H1' comprises the sequence SEQ ID NO: 11; CDR-H2' comprises the sequence SEQ ID NO: 12; and CDR-H3' comprises the sequence SEQ ID NO: 10; and a light chain comprising the following three CDRs, respectively CDR-L1', CDR-L2' and CDR-L3', wherein CDR-L1' comprises the sequence SEQ ID NO: 13; CDR-L2' comprises the sequence SEQ ID NO: 6; and CDR-L3' comprises the sequence SEQ ID NO: 7;
and a chemotherapeutic agent, preferably cisplatin, for their combined use in treating a Head & Neck cancer expressing CK8.

3. An anti-CK8 monoclonal antibody comprising a heavy chain comprising the following three CDRs, respectively CDR-H1, CDR-H2 and CDR-H3, wherein CDR-H1 comprises the sequence SEQ ID NO: 49; CDR-H2 comprises the sequence SEQ ID NO: 50; and CDR-H3 comprises the sequence SEQ ID NO: 51; and a light chain comprising the following three CDRs, respectively CDR-L1, CDR-L2 and CDR-L3, wherein CDR-L1 comprises the sequence SEQ ID NO: 52; CDR-L2 comprises the sequence SEQ ID NO: 47; and CDR-L3 comprises the sequence SEQ ID NO: 53.

4. The antibody of claim 3, having the VH sequence SEQ ID NO: 38 and the VL sequence SEQ ID NO: 36.

5. The antibody of claim 3 or 4, further comprising the Heavy constant domain of SEQ ID NO: 18 and/or the Light constant region (kappa) of SEQ ID NO: 20.

6. The antibody of any one of claims 3 to 5, for its use in treating a tumour expressing CK8, in particular colorectal (CRC), Head & Neck (HAN), Glioblastoma (GBM), Urinary (U), Prostate (PC), Breast (B), Lung (L), Pancreatic (PRC) or Renal (R), expressing CK8.

7. An anti-CK8 monoclonal antibody comprising a heavy chain comprising the following three CDRs, respectively CDR-H1, CDR-H2 and CDR-H3, wherein CDR-H1 comprises the sequence SEQ ID NO: 43; CDR-H2 comprises the sequence SEQ ID NO: 44; and CDR-H3 comprises the sequence SEQ ID NO: 45; and a light chain comprising the following three CDRs, respectively CDR-L1, CDR-L2 and CDR-L3, wherein CDR-L1 comprises the sequence SEQ ID NO: 46; CDR-L2 comprises the sequence SEQ ID NO: 47; and CDR-L3 comprises the sequence SEQ ID NO: 48.

8. The antibody of claim 7, having the VH sequence SEQ ID NO: 42 and the VL sequence SEQ ID NO: 40.

9. The antibody of claim 7 or 8, further comprising the Heavy constant domain of SEQ ID NO: 18 and/or the Light constant region (kappa) of SEQ ID NO: 20.

10. The antibody of any one of claims 7 to 9, for its use in treating a tumour expressing CK8, in particular colorectal (CRC), Head & Neck (HAN), Glioblastoma (GBM), Urinary (U), Prostate (PC), Breast (B), Lung (L), Pancreatic (PRC) or Renal (R), expressing CK8.

11. A pharmaceutical composition comprising an antibody according to any one of claims 3 to 5 and 7 to 9, and a pharmaceutically acceptable vehicle.

12. The composition of claim 11, for its use in a CK-8 expressing solid cancer.
